# FASCICULE DE BREVET EUROPEEN

(11) **EP 0 821 680 B1**
(45) Date de publication et mention de la délivrance du brevet: **30.08.2000**
(21) Numéro de dépôt: 96911009.7
(22) Date de dépôt: 01.04.1996
(51) Int. Cl.: C07D 305/14, C07D 405/12, C07D 407/12, C07D 409/12, A61K 31/335

(54) **NOUVEAUX TAXOIDES, LEUR PREPARATION ET LES COMPOSITIONS PHARMACEUTIQUES QUI LES CONTIENNENT**
TAXOL DERIVATE,DEREN HERSTELLUNG UND DIESE ENTHALTENDE PHARMAZEUTISCHE ZUSAMMENSTELLUNGEN
NOVEL TAXOIDS, PREPARATION THEREOF, AND PHARMACEUTICAL COMPOSITIONS CONTAINING SAME

(30) Priorité: 03.04.1995 FR 9503868
(43) Date de publication de la demande: 04.02.1998
(73) Titulaire: Aventis Pharma S.A., 92160 Antony (FR)
(72) Inventeur: BOUCHARD, Hervé, F-94320 Thiais (FR); BOURZAT, Jean-Dominique, F-94300 Vincennes (FR); COMMERCON, Alain, F-94400 Vitry-sur-Seine (FR)
(74) Mandataire: Le Pennec, Magali
(86) Numéro de dépôt international: FR9600487
(87) Numéro de publication internationale: WO9631493

(56) Documents cités:
- FR-A- 2 698 871
- US-A- 5 254 580

## Description

La présente invention concerne de nouveaux taxoïdes de formule générale : 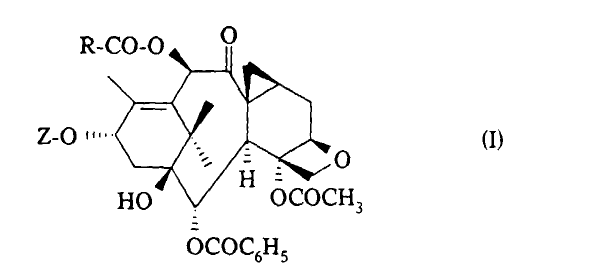 dans laquelle :
R représente
- un radical alcoyle droit ou ramifié contenant 1 à 8 atomes de carbone substitué par un atome d'halogène choisi parmi les atomes de fluor, de chlore, de brome et d'iode ou par un radical amino, alcoylamino dont la partie alcoyle contient 1 à 4 atomes de carbone, dialcoylamino dont chaque partie alcoyle contient 1 à 4 atomes de carbone ou forme avec l'atome d'azote auquel elle est liée un radical hétérocyclique saturé contenant 5 ou 6 chaînons et contenant éventuellement un second hétéroatome choisi parmi les atomes d'oxygène, de soufre ou d'azote (éventuellement substitué par un radical alcoyle contenant 1 à 4 atomes de carbone ou un radical phényle ou un radical phénylalcoyle dont la partie alcoyle contient 1 à 4 atomes de carbone), carboxy, alcoyloxycarbonyle dont la partie alcoyle contient 1 à 4 atomes de carbone, cyano, carbamoyle, N-alcoylcarbamoyle dont la partie alcoyle contient 1 à 4 atomes de carbone ou N,N-dialcoylcarbamoyle dont chaque partie alcoyle contient 1 à 4 atomes de carbone ou forme avec l'atome d'azote auquel elle est liée un radical hétérocyclique saturé contenant 5 ou 6 chaînons et éventuellement un second hétéroatome choisi parmi les atomes d'oxygène, de soufre ou d'azote (éventuellement substitué par un radical alcoyle contenant 1 à 4 atomes de carbone ou un radical phényle ou un radical phénylalcoyle dont la partie alcoyle contient 1 à 4 atomes de carbone),
- un radical alcényle contenant 2 à 8 atomes de carbone en chaîne droite ou ramifiée, un radical alcynyle contenant 2 à 8 atomes de carbone en chaîne droite ou ramifiée, un radical cycloalcoyle contenant 3 à 6 atomes de carbone, cycloalcényle contenant 3 à 6 atomes de carbone, ces radicaux étant éventuellement substitués par un atome d'halogène choisi parmi les atomes de fluor, de chlore, de brome et d'iode ou par un radical amino, alcoylamino dont la partie alcoyle contient 1 à 4 atomes de carbone, dialcoylamino dont chaque partie alcoyle contient 1 à 4 atomes de carbone ou forme avec l'atome d'azote auquel elle est liée un radical hétérocyclique saturé contenant 5 ou 6 chaînons et éventuellement un second hétéroatome choisi parmi les atomes d'oxygène, de soufre ou d'azote (éventuellement substitué par un radical alcoyle contenant 1 à 4 atomes de carbone ou un radical phényle ou un radical phénylalcoyle dont la partie alcoyle contient 1 à 4 atomes de carbone), carboxy, alcoyloxycarbonyle dont la partie alcoyle contient 1 à 4 atomes de carbone, cyano, carbamoyle, N-alcoylcarbamoyle dont la partie alcoyle contient 1 à 4 atomes de carbone ou N,N-dialcoylcarbamoyle dont chaque partie alcoyle contient 1 à 4 atomes de carbone ou forme avec l'atome d'azote auquel elle est liée un radical hétérocyclique saturé contenant 5 ou 6 chaînons et éventuellement un second hétéroatome choisi parmi les atomes d'oxygène, de soufre ou d'azote (éventuellement substitué par un radical alcoyle contenant 1 à 4 atomes de carbone ou un radical phényle ou un radical phénylalcoyle dont la partie alcoyle contient 1 à 4 atomes de carbone), ou
- un radical phényle ou hétérocyclique aromatique à 5 ou 6 chaînons contenant comme hétéroatome un atome d'oxygène, de soufre ou d'azote, et

Z représente un atome d'hydrogène ou un radical de formule générale : 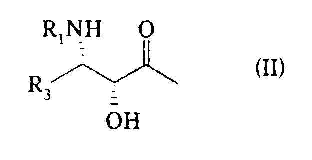 dans laquelle :
R₁ représente un radical benzoyle éventuellement substitué par un ou plusieurs atomes ou radicaux, identiques ou différents, choisis parmi les atomes d'halogène et les radicaux alcoyles contenant 1 à 4 atomes de carbone, alcoxy contenant 1 à 4 atomes de carbone ou trifluorométhyle, thénoyle ou furoyle ou un radical R₂-O-CO- dans lequel R₂ représente :
   - un radical alcoyle contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone, bicycloalcoyle contenant 7 à 10 atomes de carbone, ces radicaux étant éventuellement substitués par un ou plusieurs substituants choisis parmi les atomes d'halogène et les radicaux hydroxy, alcoxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle (éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone), cyano, carboxy ou alcoxycarbonyle dont la partie alcoyle contient 1 à 4 atomes de carbone,
   - un radical phényle ou α- ou β-naphtyle éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone,
   - ou un radical hétérocyclique aromatique à 5 chaînons choisi de préférence parmi les radicaux furyle et thiényle,
   - ou un radical hétérocyclyle saturé contenant 4 à 6 atomes de carbone éventuellement substitué par un ou plusieurs radicaux alcoyles contenant 1 à 4 atomes de carbone,
R₃ représente un radical alcoyle droit ou ramifié contenant 1 à 8 atomes de carbone, alcényle droit ou ramifié contenant 2 à 8 atomes de carbone, alcynyle droit ou ramifié contenant 2 à 8 atomes de carbone, cycloalcoyle contenant 3 à 6 atomes de carbone, phényle ou α- ou β-naphtyle éventuellement substitué par un ou plusieurs atomes ou radicaux choisis parmi les atomes d'halogène et les radicaux alcoyles, alcényles, alcynyles, aryles, aralcoyles, alcoxy, alcoylthio, aryloxy, arylthio, hydroxy, hydroxyalcoyle, mercapto, formyle, acyle, acylamino, aroylamino, alcoxycarbonylamino, amino, alcoylamino, dialcoylamino, carboxy, alcoxycarbonyle, carbamoyle, alcoylcarbamoyle, dialcoylcarbamoyle, cyano, nitro et trifluorométhyle, ou un hétérocycle aromatique ayant 5 ou 6 chaînons et contenant un ou plusieurs hétéroatomes, identiques ou différents, choisis parmi les atomes d'azote, d'oxygène ou de soufre et éventuellement substitué par un ou plusieurs substituants, identiques ou différents, choisis parmi les atomes d'halogène et les radicaux alcoyles, alcoxy, aryles, aryloxy, amino, alcoylamino, dialcoylamino, acylamino, alcoxycarbonylamino, acyle, arylcarbonyle, cyano, carboxy, carbamoyle, alcoyl-carbamoyle, dialcoylcarbamoyle ou alcoxycarbonyle, étant entendu que, dans les substituants des radicaux phényle, α- ou (3-naphtyle et hétérocyclyles aromatiques, les radicaux alcoyles et les portions alcoyles des autres radicaux contiennent 1 à 4 atomes de carbone et que les radicaux alcényles et alcynyles contiennent 2 à 8 atomes de carbone et que les radicaux aryles sont des radicaux phényles ou α- ou β-naphtyles.

Parmi les composés décrits dans l'art antérieur on peut citer le brevet FR 2 698 871 qui décrit des composés porteurs d'un groupe cyclopropyl en position 7-8 et porteurs en position 10 soit d'un groupe hydroxyle soit d'un groupe acétyle, les autres substituants étant similaires à ceux de la présente invention.

Le brevet US 5 254 580 décrit des composés porteurs d'un groupe cyclopropyl en position 7-8 et porteurs en position 10 d'un groupe -OCOR, -OCOOR, H, OH ou CO. Ces dérivés sont tous différents des dérivés de la présente invention.

De préférence les radicaux aryles pouvant être représentés par R₃ sont des radicaux phényles ou α- ou β-naphtyles éventuellement substitués par un ou plusieurs atomes ou radicaux choisis parmi les atomes d'halogène (fluor, chlore, brome, iode) et les radicaux alcoyles, alcényles, alcynyles, aryles, arylalcoyles, alcoxy, alcoylthio, aryloxy, arylthio, hydroxy, hydroxyalcoyle, mercapto, formyle, acyle, acylamino, aroylamino, alcoxycarbonylamino, amino, alcoylamino, dialcoylamino, carboxy, alcoxycarbonyle, carbamoyle, alcoylcarbamoyle dialcoylcarbamoyle, cyano, nitro et trifluorométhyle, étant entendu que les radicaux alcoyles et les portions alcoyles des autres radicaux contiennent 1 à 4 atomes de carbone, que les radicaux alcényles et alcynyles contiennent 2 à 8 atomes de carbone et que les radicaux aryles sont des radicaux phényles ou α- ou β-naphtyles.

De préférence les radicaux hétérocycliques pouvant être représentés par R₃ sont des radicaux hétérocycliques aromatiques ayant 5 ou 6 chaînons et contenant un ou plusieurs atomes, identiques ou différents, choisis parmi les atomes d'azote, d'oxygène ou de soufre, éventuellement substitués par un ou plusieurs substituants, identiques ou différents, choisis parmi les atomes d'halogène (fluor, chlore, brome, iode) et les radicaux alcoyles contenant 1 à 4 atomes de carbone, aryles contenant 6 à 10 atomes de carbone, alcoxy contenant 1 à 4 atomes de carbone, aryloxy contenant 6 à 10 atomes de carbone, amino, alcoylamino contenant 1 à 4 atomes de carbone, dialcoylamino dont chaque partie alcoyle contient 1 à 4 atomes de carbone, acylamino dont la partie acyle contient 1 à 4 atomes de carbone, alcoxycarbonylamino contenant 1 à 4 atomes de carbone, acyle contenant 1 à 4 atomes de carbone, arylcarbonyle dont la partie aryle contient 6 à 10 atomes de carbone, cyano, carboxy, carbamoyle, alcoylcarbamoyle dont la partie alcoyle contient 1 à 4 atomes de carbone, dialcoylcarbamoyle dont chaque partie alcoyle contient 1 à 4 atomes de carbone ou alcoxycarbonyle dont la partie alcoxy contient 1 à 4 atomes de carbone.

De préférence, R représente un radical alcoyle contenant 1 à 8 atomes de carbone substitué par un atome d'halogène ou par un radical diméthylamino, diéthylamino, carboxy, méthoxycarbonyle, éthoxycarbonyle, cyano, carbamoyle, N-méthylcarbamoyle, N,N-diméthylcarbamoyle, pyrrolidinocarbonyle ou pipéridinocarbonyle, un radical alcényle contenant 2 à 8 atomes de carbone, alcynyle contenant 2 à 8 atomes de carbone, cycloalcoyle contenant 3 à 6 atomes de carbone ou cycloalcényle contenant 3 à 6 atomes de carbone éventuellement substitué par un atome d'halogène ou par un radical diméthylamino, diéthylamino, carboxy, méthoxycarbonyle, éthoxy-carbonyle, cyano, carbamoyle, N-méthylcarbamoyle, N,N-diméthylcarbamoyle, pyrroli-dinocarbonyle ou pipéridinocarbonyle, phényle, pyridyl-2, pyridyl-3, pyridyl-4, thiényl-2, thiényl-3, furyl-2, furyl-3, thiazolyl-2, -4 ou -5.

Plus particulièrement, l'invention concerne les produits de formule générale (I) dans laquelle Z représente un atome d'hydrogène ou un radical de formule générale (II) dans laquelle R₁ représente un radical benzoyle ou un radical R₂-O-CO- dans lequel R₂ représente un radical tert-butyle et R₃ représente un radical alcoyle contenant 1 à 6 atomes de carbone, alcényle contenant 2 à 6 atomes de carbone, cycloalcoyle contenant 3 à 6 atomes de carbone, phényle éventuellement substitué par un ou plusieurs atomes ou radicaux, identiques ou différents choisis parmi les atomes d'halogène (fluor, chlore) et les radicaux alcoyles (méthyle), alcoxy (méthoxy), dialcoylamino (diméthylamino), acylamino (acétylamino), alcoxycarbonylamino (tert-butoxycarbonylamino) ou trifluorométhyle ou un radical furyle-2 ou -3, thiényle-2 ou -3 ou thiazolyle-2, -4 ou -5 et R représente un radical cycloalcoyle contenant 3 à 6 atomes de carbone ou un radical phényle, pyridyl-2, -3 ou -4, furyl-2 ou -3 et thiényl- 2 ou -3.

Plus particulièrement encore, l'invention concerne les produits de formule générale (I) dans laquelle Z représente un atome d'hydrogène ou un radical de formule générale (II) dans laquelle R₁ représente un radical benzoyle ou un radical R₂-O-CO- dans lequel R₂ représente un radical tert-butyle et R₃ représente un radical isobutyle, isobutényle, butényle, cyclohexyle, phényle, furyl-2, furyl-3, thiényl-2, thiényl-3, thiazolyl-2, thiazolyl-4 ou thiazolyl-5, R représente un radical cyclopropyle, cyclopentyle, phényle, pyridyl-2, thiényl-2 ou furyl-2.

Les produits de formule générale (I) dans laquelle Z représente un radical de formule générale (II) présentent des propriétés antitumorales et antileucémiques remarquables.

Selon l'invention, les produits de formule générale (I) dans laquelle Z représente un radical de formule générale (II) peuvent être obtenus par estérification d'un produit de formule générale : 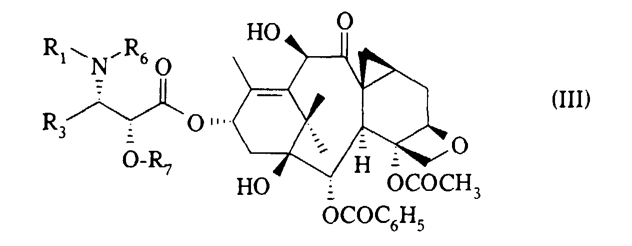 dans laquelle R₁ et R₃ sont définis comme précédemment, ou bien R₆ représente un atome d'hydrogène et R₇ représente un groupement protecteur de la fonction hydroxy, et ou bien R₆ et R₇ forment ensemble un hétérocycle saturé à 5 ou 6 chaînons, au moyen d'un acide de formule générale :

R-CO-OH (IV)

dans laquelle R est défini comme précédemment, ou d'un dérivé de cet acide tel qu'un halogénure, l'anhydride symétrique ou un anhydride mixte, pour obtenir un produit de formule générale : 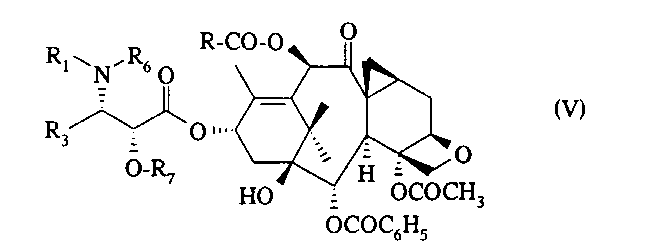 dans laquelle R₁, R₃, R₆ et R₇ sont définis comme précédemment, suivie du remplacement des groupements protecteurs R₇ ou R₆ et R₇ par des atomes d'hydrogène.

L'estérification au moyen d'un acide de formule générale (IV) peut être effectuée en présence d'un agent de condensation (carbodiimide, carbonate réactif) et d'un agent d'activation (aminopyridines) dans un solvant organique (éther, ester, cétones, nitriles, hydrocarbures aliphatiques, hydrocarbures aliphatiques halogénés, hydrocarbures aromatiques) à une température comprise entre -10 et 90°C.

L'estérification peut aussi être réalisée en utilisant l'acide de formule générale (IV) sous forme d'anhydride symétrique en opérant en présence d'un agent d'activation (aminopyridines) dans un solvant organique (éthers, esters, cétones, nitriles, hydrocarbures aliphatiques, hydrocarbures aliphatiques halogénés, hydrocarbures aromatiques) à une température comprise entre 0 et 90°C.

L'estérification peut aussi être réalisée en utilisant l'acide de formule générale (IV) sous forme d'halogénure ou sous forme d'anhydride mixte avec un acide aliphatique ou aromatique, éventuellement préparé in situ, en présence d'une base (amine aliphatique tertiaire) en opérant dans un solvant organique (éthers, esters, cétones, nitriles, hydrocarbures aliphatiques, hydrocarbures aliphatiques halogénés, hydrocarbures aromatiques) à une température comprise entre 0 et 80°C.

Lorsque R₆ représente un atome d'hydrogène, R₇ représente de préférence un radical méthoxyméthyle, éthoxy-1 éthyle, benzyloxyméthyle, triméthylsilyle, triéthylsilyle, β-triméthylsilyléthoxyméthyle, benzyloxycarbonyle ou tétrahydropyrannyle.

Lorsque R₆ et R₇ forment ensemble un hétérocycle, celui-ci est de préférence un cycle oxazolidine éventuellement mono-substitué ou gem-disubstitué en position -2.

Le remplacement des groupements protecteurs R₇ et/ou R₆ et R₇ par des atomes d'hydrogène peut être effectué, selon leur nature de la manière suivante :
1) lorsque R₆ représente un atome d'hydrogène et R₇ représente un groupement protecteur de la fonction hydroxy, le remplacement des groupements protecteurs par des atomes d'hydrogène s'effectue au moyen d'un acide minéral (acide chlorhydrique, acide sulfurique, acide fluorhydrique) ou organique (acide acétique, acide méthanesulfonique, acide trifluorométhanesulfonique, acide p.toluènesulfonique) utilisé seul ou en mélange en opérant dans un solvant organique choisi parmi les alcools, les éthers, les esters, les hydrocarbures aliphatiques, les hydrocarbures aliphatiques halogénés, les hydrocarbures aromatiques ou les nitriles à une température comprise entre -10 et 60°C,
2) lorsque R₆ et R₇ forment ensemble un hétérocycle saturé à 5 ou 6 chaînons et plus particulièrement un cycle oxazolidine de formule générale : 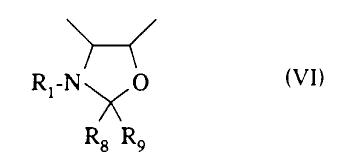 dans laquelle R₁ est défini comme précédemment, R₈ et R₉, identiques ou différents, représentent un atome d'hydrogène ou un radical alcoyle contenant 1 à 4 atomes de carbone, ou un radical aralcoyle dont la partie alcoyle contient 1 à 4 atomes de carbone et la partie aryle représente, de préférence, un radical phényle éventuellement substitué par un ou plusieurs radicaux alcoxy contenant 1 à 4 atomes de carbone, ou un radical aryle représentant, de préférence un radical phényle éventuellement substitué par un ou plusieurs radicaux alcoxy contenant 1 à 4 atomes de carbone, ou bien R₈ représente un radical alcoxy contenant 1 à 4 atomes de carbone ou un radical trihalométhyle tel que trichlorométhyle ou un radical phényle substitué par un radical trihalométhyle tel que trichlorométhyle et R₉ représente un atome d'hydrogène, ou bien R₈ et R₉ forment ensemble avec l'atome de carbone auquel ils sont liés un cycle ayant 4 à 7 chaînons, le remplacement du groupement protecteur formé par R₆ et R₇ par des atomes d'hydrogène peut être effectué, selon les significations de R₁, R₈ et R₉, de la manière suivante :
   a) lorsque R₁ représente un radical tert-butoxycarbonyle, R₈ et R₉, identiques ou différents, représentent un atome d'hydrogène ou un radical alcoyle ou un radical aralcoyle (benzyle) ou aryle (phényle), ou bien R₈ représente un radical trihalométhyle ou un radical phényle substitué par un radical trihalométhyle, et R₉ représente un atome d'hydrogène, ou bien R₈ et R₉ forment ensemble un cycle ayant de 4 à 7 chaînons, le traitement de l'ester de formule générale (V) par un acide minéral ou organique éventuellement dans un solvant organique tel qu'un alcool conduit au produit de formule générale : 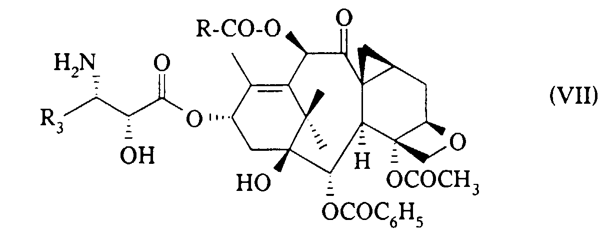 dans laquelle R₃ est défini comme précédemment, qui est acylé au moyen de chlorure de benzoyle dans lequel le noyau phényle est éventuellement substitué, de chlorure de thénoyle, de chlorure de furoyle ou d'un produit de formule générale :

      R₂-O-CO-X (VIII)

      dans laquelle R₂ est défini comme précédemment et X représente un atome d'halogène (fluor, chlore) ou un reste -O-R₂ ou -O-CO-O-R₂, pour obtenir un produit de formule générale (I) dans laquelle Z représente un radical de formule générale (II).
      De préférence, le produit de formule générale (V) est traité par l'acide formique à une température voisine de 20°C pour fournir le produit de formule générale (VII).
      De préférence, l'acylation du produit de formule générale (VII) au moyen d'un chlorure de benzoyle dans lequel le radical phényle est éventuellement substitué, de chlorure de thénoyle ou de chlorure de furoyle ou d'un produit de formule générale (VIII) est effectuée dans un solvant organique inerte choisi parmi les esters tels que l'acétate d'éthyle, l'acétate d'isopropyle ou l'acétate de n.butyle et les hydrocarbures aliphatiques halogénés tels que le dichlorométhane ou le dichloro-1,2 éthane en présence d'une base minérale telle que le bicarbonate de sodium ou organique telle que la triéthylamine. La réaction est effectuée à une température comprise entre 0 et 50°C, de préférence voisine de 20°C.
   b) lorsque R₁ représente un radical benzoyle éventuellement substitué, thénoyle ou furoyle ou un radical R₂O-CO- dans lequel R₂ est défini comme précédemment, R₈ représente un atome d'hydrogène ou un radical alcoxy contenant 1 à 4 atomes de carbone ou un radical phényle substitué par un ou plusieurs radicaux alcoxy contenant 1 à 4 atomes de carbone et R₉ représente un atome d'hydrogène, le remplacement du groupement protecteur formé par R₆ et R₇ par des atomes d'hydrogène s'effectue en présence d'un acide minéral (acide chlorhydrique, acide sulfurique) ou organique (acide acétique, acide méthanesulfonique, acide trifluorométhanesulfonique, acide p.toluène-sulfonique) utilisé seul ou en mélange en quantité stoechiométrique ou catalytique, en opérant dans un solvant organique choisi parmi les alcools, les éthers, les esters, les hydrocarbures aliphatiques, les hydrocarbures aliphatiques halogénés et les hydrocarbures aromatiques à une température comprise entre -10 et 60°C, de préférence entre 15 et 30°C.

Les produits de formule générale (III) peuvent être obtenus dans les conditions décrites dans la demande internationale PCT WO 94/13654.

Les produits de formule générale (III) peuvent aussi être obtenus par estérification d'un dérivé de la baccatine III de formule : 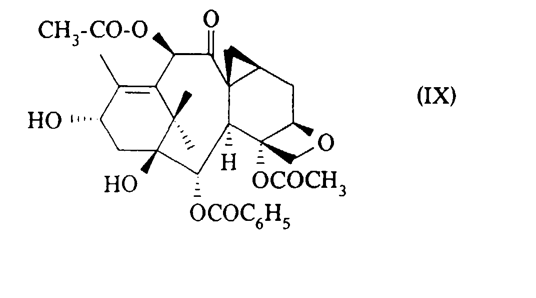 au moyen d'un acide de formule générale : 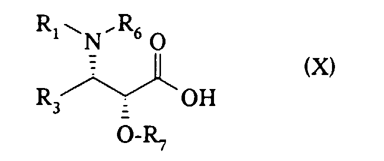 dans laquelle R₁, R₃, R₆ et R₇ sont définis comme précédemment, ou d'un dérivé de cet acide tel qu'un halogénure, l'anhydride symétrique ou un anhydride mixte, puis remplace le radical acétoxy en 10 par un radical hydroxy.

L'estérification s'effectue dans des conditions analogues à celles décrites précédemment pour l'estérification d'un produit de formule générale (III) au moyen d'un acide de formule générale (IV).

Le remplacement du radical acétoxy en 10 par un radical hydroxy s'effectue généralement au moyen d'iodure de zinc.

Le produit de formule (IX) peut être obtenu dans les conditions décrites dans la demande internationale PCT WO 94/13654 par action d'un halogénure de métal alcalin (iodure de sodium, fluorure de potassium) ou d'un azoture de métal alcalin (azoture de sodium) ou d'un sel d'ammonium quaternaire ou d'un phosphate de métal alcalin sur le benzoyloxy-2α diacetoxy-4α,10β dihydroxy-1β,13α époxy-5β,20 trifluorométhyl-sulfonyloxy-7β oxo-9 taxène-11.

Les produits de formule générale (I) dans laquelle Z représente un radical de formule générale (II) peuvent aussi être obtenus par estérification d'un produit de formule générale : 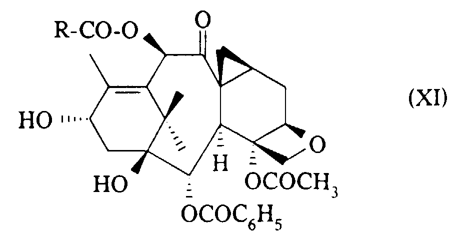 dans laquelle R est défini comme précédemment, au moyen d'un acide de formule générale (X), ou d'un dérivé de cet acide tel qu'un halogénure, l'anhydride symétrique ou un anhydride mixte, pour obtenir un produit de formule générale (V) dont les groupements protecteurs R₇ ou R₆ et R₇ sont remplacés par des atomes d'hydrogène dans les conditions décrites précédemment.

Les produits de formule générale (XI), c'est-à-dire les produits de formule générale (I) dans laquelle Z représente un atome d'hydrogène peuvent être obtenus par estérification d'un produit de formule : 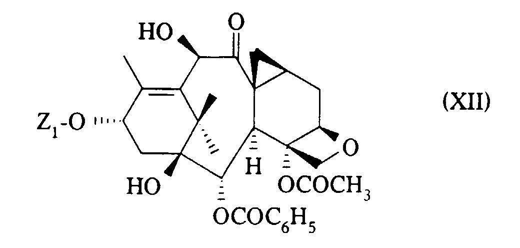 dans laquelle Z₁ représente un groupement protecteur de la fonction hydroxy tel qu'un radical silylé comme le radical triéthylsilyle, au moyen d'un acide de formule générale (IV), ou d'un dérivé de cet acide tel qu'un halogénure ou l'anhydride symétrique ou un anhydride mixte dans les conditions décrites précédemment pour l'estérification d'un produit de formule générale (III) au moyen d'un acide de formule générale (IV) ou d'un dérivé de cet acide, suivi du remplacement du groupement protecteur Z₁ par un atome d'hydrogène dans des conditions qui ne touchent pas au reste de la molécule.

Le produit de formule générale (XII) peut être obtenu dans les conditions décrites dans la demande internationale PCT WO 94/13654.

Les nouveaux produits de formule générale (I) obtenus par la mise en oeuvre des procédés selon l'invention peuvent être purifiés selon les méthodes connues telles que la cristallisation ou la chromatographie.

Les produits de formule générale (I) dans laquelle Z représente un radical de formule générale (II) présentent des propriétés biologiques remarquables.

In vitro, la mesure de l'activité biologique est effectuée sur la tubuline extraite de cerveau de porc par la méthode de M.L. Shelanski et coll., Proc. Natl. Acad. Sci. USA, 70, 765-768 (1973). L'étude de la dépolymérisation des microtubules en tubuline est effectuée selon la méthode de G. Chauvière et coll., C.R. Acad. Sci., 293, série II, 501-503 (1981). Dans cette étude les produits de formule générale (I) dans laquelle Z représente un radical de formule générale (II) se sont montrés au moins aussi actifs que le taxol et le Taxotère.

In vivo, les produits de formule générale (I) dans laquelle Z représente un radical de formule générale (II) se sont montrés actifs chez la souris greffée par le mélanome B16 à des doses comprises entre 1 et 10 mg/kg par voie intrapéritonéale, ainsi que sur d'autres tumeurs liquides ou solides.

Les nouveaux produits ont des propriétés anti-tumorales et plus particulièrement une activité sur les tumeurs qui sont résistantes au Taxol® ou au Taxotère®. De telles tumeurs comprennent les tumeurs du colon qui ont une expression élevée du gène mdr 1 (gène de la multi-drug resistance). La multi-drug resistance est un terme habituel se rapportant à la résistance d'une tumeur à différents produits de structures et de mécanismes d'action différents. Les taxoïdes sont généralement connus pour être fortement reconnus par des tumeurs expérimentales telles que P388/DOX, une lignée cellulaire sélectionnée pour sa résistance à la doxorubicine (DOX) et qui surexprime mdr 1.

Les exemples suivants illustrent la présente invention.

### EXEMPLE 1

A une solution de 62 mg d'acide pyridine-2 carboxylique dans 25 cm3 d'acétate d'éthyle anhydre, maintenue sous atmosphère d'argon et sous agitation, on ajoute, à une température voisine de 20°C, 380 mg de tert-butoxycarbonyl-3 (méthoxy-4 phényl)-2 phényl-4 oxazolidine-1,3 carboxylate-5(2R,4S,5R) d'acétoxy-4α benzoyloxy-2α dihydroxy-1β,10β époxy-5β,20 méthylène-7β,8β oxo-9 nor-19 taxène-11 yle-13α, 25 mg de diméthylamino-4 pyridine, 0,5 g de tamis moléculaire 4 Å et 151 mg de N,N'-dicyclohexylcarbodiimide. Le mélange réactionnel est maintenu sous agitation pendant 16 heures à une température voisine de 20°C puis on ajoute 20 mg d'acide pyridine-2 carboxylique, 8 mg de diméthylamino-4 pyridine, 100 mg de tamis moléculaire 4 Å et 50 mg de N,N'-dicyclohexylcarbodiimide et maintient à nouveau sous agitation pendant 4 heures. Le mélange réactionnel est filtré sur verre fritté garni de célite. Le verre fritté est lavé par 100 cm3 d'acétate d'éthyle, les filtrats sont réunis, lavés successivement par 15 cm3 d'une solution aqueuse saturée d'hydrogénocarbonate de sodium et par 5 fois 10 cm3 d'eau distillée, séchés sur sulfate de magnésium, filtrés et concentrés à sec sous pression réduite (2,7 kPa) à 40°C. On obtient 715 mg d'une meringue blanche que l'on purifie par chromatographie sur 40 g de silice (0,04-0,063 mm) contenus dans une colonne de 2 cm de diamètre [éluant : dichlorométhane-méthanol (99-1 en volumes)] en recueillant des fractions de 10 cm3. Les fractions ne contenant que le produit cherché sont réunies et concentrées à sec sous pression réduite (2,7 kPa) à 40°C. On obtient ainsi 297 mg de tert-butoxycarbonyl-3 (méthoxy-4 phényl)-2 phényl-4 oxazolidine-1,3 carboxylate-5(2R,4S,5R) d'acétoxy-4α benzoyloxy-2α époxy-5β,20 hydroxy-1β méthylène-7β,8β oxo-9 (pyridyl-2 carbonyl)oxy-10β nor-19 taxène-11 yle-13α sous forme d'une meringue blanche.

290 mg de tert-butoxycarbonyl-3 (méthoxy-4 phényl)-2 phényl-4 oxazolidine-1,3 carboxylate-5(2R,4S,5R) d'acétoxy-4α benzoyloxy-2α époxy-5β,20 hydroxy-1β méthylène-7β,8β oxo-9 (pyridyl-2 carbonyl)oxy-10β nor-19 taxène-11 yle-13α sont dissous dans 5,7 cm3 d'une solution éthanolique 0,1N d'acide chlorhydrique. La solution ainsi obtenue est agitée pendant 1 heure à une température voisine de 20°C puis concentrée à sec sous pression réduite (2,7 kPa) à une température voisine de 40°C. Le solide résiduel est dissous dans 50 cm3 de dichlorométhane et la solution obtenue est lavée successivement par 2 fois 3 cm3 d'une solution aqueuse saturée d'hydrogénocarbonate de sodium et par 3 fois 5 cm3 d'eau distillée, séchée sur sulfate de magnésium, filtrée et concentrée à sec sous pression réduite (2,7 kPa) à 40°C. On obtient 270 mg d'une meringue blanche que l'on purifie par chromatographie sur 30 g de silice (0,04-0,063 mm) contenus dans une colonne de 2 cm de diamètre [éluant : dichlorométhane-méthanol (99-1 en volumes)] en recueillant des fractions de 10 cm3. Les fractions ne contenant que le produit cherché sont réunies et concentrées à sec sous pression réduite (0,27 kPa) à 20°C pendant 16 heures. On obtient ainsi 189 mg de tert-butoxycarbonylamino-3 hydroxy-2 phényl-3 propionate-(2R,3S) d'acétoxy-4α benzoyloxy-2α époxy-5β,20 hydroxy-1β méthylène-7β,8β oxo-9 (pyridyl-2 carbonyl)oxy-10β nor-19 taxène-11 yle-13α sous forme d'une meringue blanche dont les caractéristiques sont les suivantes :
- pouvoir rotatoire : [α]^{D}₂₀ = -24 (c = 0,52 ; méthanol)
- spectre de R.M.N. ¹H (300 MHz ; CDCI₃ ; déplacements chimiques δ en ppm ; constante de couplage J en Hz) : 1,28 (s, 9H : -C(CH₃)₃) ; 1,41 (s, 3H : -CH₃ en 16 ou 17) ; 1,44 (mt, 1H : -H en 7) ; 1,57 (s, 3H : -CH₃ en 16 ou 17) ; 1,69 et 2,25 (respectivement dd et mt, J = 6 et 5,5, 1H chacun : -CH₂- en 19) ; 1,89 (s, 1H : -OH en 1) ; 1,92 (s, 3H : -CH₃); 2,11 et 2,50 (respectivement d large et dt, J = 16 et J = 16 et 4,5 Hz, 1H chacun : -CH₂- en 6) ; 2,25 et 2,39 (2 mt, 1H chacun : -CH₂- en 14) ; 2,40 (s, 3H : -COCH₃) ; 3,29 (mt, 1H: -OH en 2') ; 4,04 et 4,32 (2 d, J = 9, 1H chacun : -CH₂- en 20) ; 4,15 (d, J = 7,5, 1H: -H en 3) ; 4,62 (mt, 1H: -H en 2') ; 4,74 (d, J = 4,5, 1H: -H en 5) ; 5,28 (d large, J = 10, 1H : -H en 3') ; 5,35 (d, J = 10, 1H: -CONH-) ; 5,61 (d, J = 7,5, 1H : -H en 2) ; 6,28 (t large, J = 9, 1H: -H en 13) ; 6,64 (s, 1H : -H en 10) ; de 7,25 à 7,45 (mt, 5H : -C₆H₅ en 3') ; 7,51 [(mt, 3H : -OCOC₆H₅ (-H en 3 et H en 5) et -C₅H₄N (-H en 5)] ; 7,60 [(t, J = 7,5, 1H : -OCOC₆H₅(-H en 4)] ; 7.85 [(dt, J = 8 et 1,5, 1H : -C₅H₄N (-H en 4)] ; 8,11 [(d, J = 8, 1H : -C₅H₄N (-H en 3)] ; 8,15 [(d, J = 7,5, 2H : -OCOC₆H₅ (-H en 2 et -H en 6)]; 8,80 [(d large, J = 4,5, 1H : -C₅H₄N (-H en 6)].

Le tert-butoxycarbonyl-3 (méthoxy-4 phényl)-2 phényl-4 oxazolidine-1,3 carboxylate-5(2R,4S,5R) d'acétoxy-4α benzoyloxy-2α dihydroxy-1β,10β époxy-5β,20 méthylène-7β,8β oxo-9 nor-19 taxène-11 yle-13α peut être préparé de la manière suivante :

A une solution de 5,5 g de tert-butoxycarbonyl-3 (méthoxy-4 phényl)-2 phényl-4 oxazolidine-1,3 carboxylate-5(2R,4S,5R) de benzoyloxy-2α diacétoxy- 4α,10β époxy-5β,20 hydroxy-1β méthylène-7β,8β oxo-9 nor-19 taxène-11 yle-13α dans 200 cm3 de méthanol anhydre, maintenue sous atmosphère d'argon et sous agitation, on ajoute, à une température voisine de 20°C, 20 g de tamis moléculaire 4 Å en poudre et 9,3 g d'iodure de zinc. Le milieu réactionnel est maintenu sous agitation pendant 3 jours à une température voisine de 20°C puis on rajoute 3,72 g d'iodure de zinc et 4 g de tamis moléculaire 4 Å et continue à agiter pendant 24 heures à une température voisine de 20°C. Le mélange réactionnel est filtré sur verre fritté garni de célite. Le verre fritté est lavé par 100 cm3 de dichlorométhane et les filtrats sont réunis et versés dans 200 cm3 d'eau distillée. Le mélange biphasique est agité pendant 30 minutes puis la phase aqueuse est séparée par décantation et réextraite par 3 fois 200 cm3 de dichlorométhane. Les phases organiques sont réunies lavées par 50 cm3 d'eau distillée, séchées sur sulfate de magnésium, filtrées et concentrées à sec sous pression réduite (2,7 kPa) à 40°C. On obtient 5,3 g d'une meringue blanche que l'on purifie par chromatographie sur 160 g de silice (0,04-0,063 mm) contenus dans une colonne de 3,8 cm de diamètre [éluant : dichlorométhane-méthanol (99-1 en volumes)] en recueillant des fractions de 100 cm3. Les fractions ne contenant que le produit cherché sont réunies et concentrées à sec sous pression réduite (2,7 kPa) à 40°C. On obtient ainsi 3,7 g d'une meringue blanche que l'on repurifie par chromatographie sur 175 g de silice (0,04-0,063 mm) contenus dans une colonne de 3,8 cm de diamètre [éluant : dichlorométhane-méthanol (99,6-0,4 en volumes)] en recueillant des fractions de 50 cm3. Les fractions ne contenant que le produit cherché sont réunies et concentrées à sec sous pression réduite (2,7 kPa) à 40°C. On obtient ainsi 1,78 g de tert-butoxycarbonyl-3 (méthoxy-4 phényl)-2 phényl-4 oxazolidine-1,3 carboxylate-5 (2R,4S,5R) d'acétoxy-4α benzoyloxy-2α dihydroxy-1β,10β époxy-5β,20 méthylène-7β,8β oxo-9 nor-19 taxène-11 yle-13α sous forme d'une meringue blanche.

Le tert-butoxycarbonyl-3 (méthoxy-4 phényl)-2 phényl-4 oxazolidine-1,3 carboxylate-5(2R,4S,5R) de benzoyloxy-2α diacétoxy-4α,10β époxy-5β,20 hydroxy-1β méthylène-7β,8β oxo-9 nor-19 taxène-11 yle-13α peut être préparé de la manière suivante :

A une solution de 4,01g d'acide tert-butoxycarbonyl-3 (méthoxy-4 phényl)-2 phényl-4 oxazolidine-1,3 carboxylique-5(2R,4S,5R) dans 190 cm3 d'acétate d'éthyle anhydre, maintenue sous atmosphère d'argon et sous agitation, on ajoute, à une température voisine de 20°C, 4,75 g de benzoyloxy-2a diacétoxy-4α,10β dihydroxy-1β,13α époxy-5β,20 méthylène-7β,8β oxo-9 nor-19 taxène-11, 0,5 g de diméthylamino-4 pyridine et 3,01 g de N,N'-dicyclohexylcarbodiimide. Le mélange réactionnel est maintenu sous agitation pendant 2 heures à une température voisine de 20°C puis filtré sur verre fritté garni de célite. Le verre fritté est lavé par 2 fois 50 cm3 d'acétate d'éthyle et les filtrats sont réunis, lavés par 5 fois 50 cm3 d'eau distillée, séchés sur sulfate de magnésium, filtrés et concentrés à sec sous pression réduite (2,7 kPa) à 40°C. On obtient 9,67 g d'une meringue jaune que l'on reprend par 70 cm3 d'oxyde de diisopropyle. La suspension obtenue est agitée pendant 1 heure à une température voisine de 20°C puis filtrée sur verre fritté. Le verre fritté est lavé par 2 fois 20 cm3 d'oxyde de diisopropyle et les filtrats sont réunis et concentrés à sec sous pression réduite (2,7 kPa) à 40°C. On obtient 8,09 g d'une meringue jaune que l'on purifie par chromatographie sur 250 g de silice (0,063-0,2 mm) contenus dans une colonne de 3,8 cm de diamètre [éluant : dichlorométhane-méthanol (99-1 en volumes)] en recueillant des fractions de 100 cm3. Les fractions ne contenant que le produit cherché sont réunies et concentrées à sec sous pression réduite (2,7 kPa) à 40°C. On obtient ainsi 7,23 g de tert-butoxycarbonyl-3 (méthoxy-4 phényl)-2 phényl-4 oxazolidine-1,3 carboxylate-5 (2R,4S,5R) de benzoyloxy-2α diacétoxy-4α,10β époxy-5β,20 hydroxy-1β méthylène-7β,8β oxo-9 nor-19 taxène-11 yle-13α sous forme d'une meringue blanche.

L'acide tert-butoxycarbonyl-3 (méthoxy-4 phénol)-2 phényl-4 oxazolidine-1,3 carboxylique-5(2R,4S,5R) peut être préparé de la manière suivante :

Une solution de 10,0 g de t.butoxycarbonylamino-3 hydroxy-2 phényl-3 propionate-(2R,3S) de méthyle et de 0,25 g de p.toluènesulfonate de pyridinium dans 200 cm3 de toluène est déshydratée par distillation de 20 cm3 de solvant. On ajoute 6,34 cm3 de diméthylacétal du p.méthoxybenzaldéhyde en 5 minutes sur le mélange réactionnel chauffé à l'ébullition. Pendant l'addition, on distille 50 cm3 de solvant puis on distille encore 100 cm3 de solvant. Après refroidissement à une température voisine de 20°C, on ajoute, en 10 minutes, 80 cm3 de cyclohexane. Le mélange est refroidi à 0-5°C. La bouillie obtenue est filtrée sur verre fritté et le gâteau de filtration est lavé avec 40 cm3 de cyclohexane puis séché sous pression réduite à une température voisine de 20°C. On obtient ainsi, avec un rendement de 74 %, 10,39 g de t.butoxycarbonyl-3 (méthoxy-4 phényl)-2 phényl-4 méthoxycarbonyl-5 oxazolidine-1,3-(2R,4S,5R) dont les caractéristiques sont les suivantes :
- spectre infra-rouge (en comprimé avec KBr) : bandes d'absorption caractéristiques à 3100-3000, 2980, 2960, 2930, 2910, 2840, 1740, 1700, 1614, 1514, 1460, 1435, 1390, 1370, 1245, 1175, 1165, 816, 760 et 700 cm⁻¹
- spectre de résonance magnétique nucléaire du proton (400 MHz ; CDCl₃ ; température : 323°K ; déplacements chimiques δ en ppm ; constantes de couplage J en Hz) : 1,11 (s, 9H) ; 3,60 (s, 3H) ; 3,82 (s, 3H) ; 4,58 (d, J = 5, 1H); 5,42 (d large, J = 5, 1H); 6,38 (s large, 1H); 6,92 (d, J = 7,5, 2H) ; 7,30 à 7,45 (mt, 7H).

A une solution de 3,0 g du produit obtenu précédemment dans 27 cm3 de méthanol, on ajoute 14 cm3 d'une solution aqueuse contenant 0,31 g d'hydroxyde de lithium monohydraté. On agite pendant 2 heures à une température voisine de 20°C. Le méthanol est éliminé par distillation sous pression réduite puis on ajoute 40 cm3 de dichlorométhane. Sous forte agitation, le mélange réactionnel est acidifié par addition d'acide chlorhydrique 1N jusqu'à pH = 1. Après décantation, la phase aqueuse est extraite 2 fois par 40 cm3 de dichlorométhane. Les phases organiques réunies sont séchées sur sulfate de sodium. Après filtration et évaporation du solvant, on obtient, avec un rendement de 94,5 %, 2,88 g d'acide t.butoxycarbonyl-3 (méthoxy-4 phényl)-2 phényl-4 oxazolidine-1,3 carboxylique-5-(2R,4S,5R) dont les caractéristiques sont les suivantes :
- spectre infra-rouge (en comprimé avec KBr) : bandes d'absorption caractéristiques à 3325-2675, 2980, 2955, 2935, 2845, 1755, 1700, 1615, 1590, 1515, 1460, 1250, 1175, 1030, 835, 765 et 705 cm⁻¹
- spectre de résonance magnétique nucléaire du proton (250 MHz ; CDCl₃ ; déplacements chimiques d en ppm ; constantes de couplage J en Hz) : 1,08 (s, 9H) ; 3,82 (s, 3H) ; 4,61 (d, J = 5, 1H); 5,42 (d large, J = 5, 1H); 6,38 (s large, 1H); 6,92 (d, J = 7,5, 2H) ; 7,30 à 7,45 (mt, 7H).

Le benzoyloxy-2α diacétoxy-4α,10β, dihydroxy-1β,13α époxy-5β,20 méthylène-7β,8β oxo-9 nor-19 taxène-11 peut être préparé de la manière suivante :

A une solution de 3,85 g de benzoyloxy-2α diacétoxy-4α,10β dihydroxy-1β,13α époxy-5β,20 oxo-9 trifluorométhylsulfonyloxy-7β taxène-11 dans un mélange de 75 cm3 d'acétonitrile et de 7,5 cm3 de tétrahydrofuranne anhydre, maintenue sous atmosphère d'argon et sous agitation, on ajoute, à une température voisine de 20°C, 1,9 g de tamis moléculaire 4 Å en poudre et 5,8 g de chlorure de sodium. Le mélange réactionnel est agité pendant 30 minutes à une température voisine de 20°C puis chauffé jusqu'au reflux (75°C) et maintenu au reflux pendant 2,5 heures. Après refroidissement jusqu'à une température voisine de 20°C, le mélange réactionnel est filtré sur verre fritté. Le verre fritté est lavé par 3 fois 80 cm3 de dichlorométhane, les filtrats sont réunis, lavés successivement par 25 cm3 d'une solution aqueuse saturée d'hydrogénocarbonate de sodium et par 2 fois 25 cm3 d'eau distillée, séchés sur sulfate de magnésium, filtrés et concentrés à sec sous pression réduite (2,7 kPa) à 40°C. On obtient 3,5 g d'une meringue blanche que l'on purifie par chromatographie sur 140 g de silice (0,063-0,2 mm) contenus dans une colonne de 3,5 cm de diamètre [éluant : dichlorométhane-méthanol (99-1 en volumes)] en recueillant des fractions de 50 cm3. Les fractions ne contenant que le produit cherché sont réunies et concentrées à sec sous pression réduite (2,7 kPa) à 40°C. On obtient ainsi 2,7 g de benzoyloxy-2α diacétoxy-4α,10β, dihydroxy-1β,13α époxy-5β,20 méthylène-7β,8β oxo-9 nor-19 taxène-11 sous forme d'une meringue blanche.

Le benzoyloxy-2α diacétoxy-4α,10β dihydroxy-1β,13α époxy-5β,20 oxo-9 trifluorométhylsulfonyloxy-7β taxène-11 peut être préparé de la manière suivante :

A une solution de 0,59 g de benzoyloxy-2α diacétoxy-4α,10β époxy-5β,20 oxo-9 trihydroxy-1β,7β,13α taxène-11 (baccatine III) dans 50 cm3 de dichlorométhane, maintenue sous atmosphère d'argon et sous agitation, on ajoute, à une température voisine de 20°C, 0,32 cm3 de pyridine anhydre puis goutte à goutte, à une température voisine de 20°C, 0,25 cm3 d'anhydride trifluorométhanesulfonique. Le milieu réactionnel est ensuite chauffé au reflux (40°C) pendant 3 heures puis on rajoute 0,08 cm3 d'anhydride trifluorométhanesulfonique et poursuit le chauffage au reflux pendant 1 heure. Après refroidissement jusqu'à une température voisine de 20°C, le milieu réactionnel est versé dans un mélange de 50 cm3 de dichlorométhane et de 20 cm3 d'eau distillée. La phase organique est séparée par décantation, lavée successivement par 10 cm3 d'une solution aqueuse 1N d'acide chlorhydrique et par 2 fois 10 cm3 d'eau distillée, séchée sur sulfate de magnésium, filtrée et concentrée à sec sous pression réduite (2,7 kPa) à 40°C. On obtient 0,75 g d'une meringue blanche que l'on purifie par chromatographie sur 60 g de silice (0,063-0,2 mm) contenus dans une colonne de 2,5 cm de diamètre [éluant : dichlorométhane-méthanol (98,5-1,5 en volumes)] en recueillant des fractions de 20 cm3. Les fractions ne contenant que le produit cherché sont réunies et concentrées à sec sous pression réduite (2,7 kPa) à 40°C. On obtient ainsi 0,675 g de benzoyloxy-2α diacétoxy-4α,10β dihydroxy-1β,13α époxy-5β,20 oxo-9 trifluorométhylsulfonyloxy-7β taxène-11 sous forme d'une meringue blanche.

La benzoyloxy-2α diacétoxy-4α,10β époxy-5β,20 oxo-9 trihydroxy-1β,7β,13α taxène-11 (baccatine III) peut être préparée de la manière suivante :

A une solution de 293,9 g de désacétyl-10 baccatine III dans 2,7 litres de pyridine, on ajoute, en 1 heure 20 minutes, 182 g de chlorure de triéthylsilyle. La solution obtenue est agitée pendant 40 heures à 5°C. On ajoute alors 360 g d'anhydride acétique en maintenant la température à 5°C. La suspension obtenue est agitée pendant 48 heures à 20°C puis versée sur 40 litres d'eau glacée. Le précipité obtenu est séparé par filtration puis lavé par 8 fois 2 litres d'eau et enfin dissous dans 3 litres d'acétate d'éthyle. La phase organique est séchée sur sulfate de magnésium. Après filtration et concentration sous pression réduite, le produit obtenu est cristallisé dans l'oxyde d'isopropyle. On obtient ainsi, avec un rendement de 77 %, la triéthylsilyl-7 baccatine III dont les caractéristiques sont les suivantes :
- point de fusion : 254°C
- spectre de résonance magnétique nucléaire du proton : (400 MHz ; CDCI₃, δ en ppm) : 0,58 (mt, 6H : CH₂ éthyl) ; 0,92 (t, J = 7,5 Hz, 9H : CH₃ éthyl) ; 1,02 (s, 3H : CH₃) ; 1,18 (s, 3H : CH₃) ; 1,68 (s, 3H : CH₃) ; 1,75 (s large, 1H : OH en 1) ; 1,87 et 2,53 (2 mt, 1H chacun : CH₂ en 6) ; 2,18 (s, 6H : CH₃ et COCH₃) ; 2,27 (mt, 2H : CH₂ en14) ; 2,28 (s, 3H : COCH₃) ; 2,47 (s large, 1H : OH en 13) ; 3,88 (d, J = 7 Hz, 1H : H 3) ; 4,13 et 4,30 (2d, J = 8,5 Hz, 1H chacun : CH₂ en 20) ; 4,50 (dd, J = 11 et 7 Hz, 1H : H en 7) ; 4,81 (mt, 1H : H en 13) ; 4,95 (d large, J = 10 Hz, 1H : H en 5) ; 5,63 (d, J = 7 Hz, 1H : H 2) ; 6,46 (s, 1H: H en 10) ; 7,46 (t, J = 8,5 Hz, 2H : -OCOC₆H₅ H en méta) ; 7,60 (t, J = 8,5 Hz, 1H : -OCOC₆H₅ H en para) ; 8,10 (d, J = 8,5 Hz, 2H : -OCOC₆H₅ H en ortho).

A une solution de 350 mg de triéthylsilyl-7 baccatine III dans 3 cm3 d'acétonitrile et 2,4 cm3 de pyridine, on ajoute 2,3 g d'acide trifluoroacétique. On agite pendant 48 heures à 50°C. Après refroidissement, le mélange réactionnel est repris par 50 cm3 de chlorure de méthylène, lavé par 2 fois 5 cm3 d'eau distillée, 10 cm3 d'acide chlorhydrique N et 2 fois 5 cm3 d'eau distillée et séché sur sulfate de magnésium. Apès filtration et concentration à sec sous pression réduite, on obtient 330 mg d'un produit qui est purifié par chromatographie sur 30 g de silice contenus dans une colonne de 2 cm de diamètre en éluant par un mélange chlorure de méthylène-méthanol (99-1 en volumes). Les 300 premiers cm3 élués sont éliminés. Les 275 cm3 suivants fournissent, après concentration à sec, 235 mg de baccatine III sous forme d'une meringue blanche. Le rendement est de 83 %.

### EXEMPLE 2

En opérant comme dans l'exemple 1, mais à partir de 270 mg de tert-butoxycarbonyl-3 (méthoxy-4 phényl)-2 phényl-4 oxazolidine-1,3 carboxylate-5 (2R,4S,5R) d'acétoxy-4α benzoyloxy-2α dihydroxy-1β,10β époxy-5β,20 méthylène-7β,8β oxo-9 nor-19 taxène-11 yle-13α, et de 46 mg d'acide thiophène-2 carboxylique, on obtient 230 mg de tert-butoxycarbonyl-3 (méthoxy-4 phényl)-2 phényl-4 oxazolidine-1,3 carboxylate-5(2R,4S,5R) d'acétoxy-4α benzoyloxy-2α époxy-5β,20 hydroxy-1β méthylène-7β,8β oxo-9 (thiényl-2 carbonyl)oxy-10β nor-19 taxène-11 yle-13α sous forme d'une meringue blanche.

En opérant comme dans l'exemple 1, mais à partir de 225 mg de tert-butoxycarbonyl-3 (méthoxy-4 phényl)-2 phényl-4 oxazolidine-1,3 carboxylate-5 (2R,4S,5R) d'acétoxy-4α benzoyloxy-2α époxy-5β,20 hydroxy-1β méthylène-7β,8β oxo-9 (thiényl-2 carbonyl)oxy-10β nor-19 taxène-11 yle-13α, on obtient 151 mg de tert-butoxycarbonylamino-3 hydroxy-2 phényl-3 propionate-(2R,3S) d'acétoxy-4α benzoyloxy-2α époxy-5β,20 hydroxy-1β méthylène-7β,8β oxo-9 (thiényl-2 carbonyl)oxy-10β nor-19 taxène-11 yle-13α sous forme d'une meringue blanche dont les caractéristiques sont les suivantes :
- pouvoir rotatoire : [α]^{D}₂₀ = - 23 (c = 0,5 ; méthanol)
- spectre de R.M.N. ¹H (300 MHz ; CDCI₃ ; déplacements chimiques δ en ppm ; constantes de couplage J en Hz) : 1,27 (s, 9H : -C(CH₃)₃) ; 1,32 (s, 3H : -CH₃ en 16 ou en 17) ; 1,39 (s, 3H : -CH₃ en 16 ou en17) ; 1,43 (mt, 1H : -H en 7) ; 1,70 et 2,27 (respectivement dd et mt, J = 6,5 et 5, 1H chacun : -CH₂- en 19) ; 1,89 (s, 1H : -OH en 1) ; 1,91 (s, 3H : CH₃) ; 2,13 et 2,48 (respectivement d large et dt, J = 16 et J = 16 et 4,5, 1H chacun : -CH₂- en 6) ; 2,25 et 2,39 (respectivement dd et mt, J = 15,5 et 9, 1H chacun : -CH₂- en 14) ; 2,39 (s, 3H : -COCH₃) ; 3,28 (mt, 1H : -OH en 2') ; 4,05 et 4,33 (2 d, J = 9, 1H chacun : -CH₂- en 20) ; 4,14 (d, J = 7,5, 1H : -H en 3) ; 4,62 (mt, 1H : -H en 2') ; 4,75 (d, J = 4,5, 1H : -H en 5) ; 5,28 (d large, J = 10, 1H : -H en 3') ; 5,37 (d, J = 10, 1H : -CONH-) ; 5,72 (d, J = 7,5, 1H : -H en 2) 6,30 (t large, J = 9, 1H : -H en 13) ; 6,52 (s, 1H : -H en 10) ; 7,15 [(dd, J = 5 et 3,5, 1H : -C₄H₃S (-H en 4)] ; de 7,25 à 7,45 (mt, 5H : -C₆H₅ en 3') ; 7,51 [(t, J = 7,5, 2H : -OCOC₆H₅ (-H en 3 et H en 5)] ; 7,61 [(t, J = 7,5, 1H : -OCOC₆H₅ (-H en 4)] ; 7,62 [(dd, J = 5 et 1,5, 1H : -C₄H₃S (-H en 5)] ; 7,88 [(dd, J = 3,5 et 1,5, 1H : -C₄H₃S (-H en 3)] ; 8,15 (d, J = 7,5, 2H : -OCOC₆H₅ (-H en 2 et H en 6).

### EXEMPLE 3

En opérant comme dans l'exemple 1, mais à partir de 270 mg de tert-butoxycarbonyl-3 (méthoxy-4 phényl)-2 phényl-4 oxazolidine-1,3 carboxylate-5 (2R,4S,5R) d'acétoxy-4α benzoyloxy-2α dihydroxy-1β,10β époxy-5β,20 méthylène-7β,8β oxo-9 nor-19 taxène-11 yle-13α, et de 0,038 cm3 d'acide cyclopentanecarboxylique, on obtient 187 mg de tert-butoxycarbonyl-3 (méthoxy-4 phényl)-2 phényl-4 oxazolidine-1,3 carboxylate-5 (2R,4S,5R) d'acétoxy-4α benzoyloxy-2α cyclopentylcarbonyloxy-10β époxy-5β,20 hydroxy-1β méthylène-7β,8β oxo-9 nor-19 taxène-11 yle-13α sous forme d'une meringue blanche.

En opérant comme dans l'exemple 1, mais à partir de 182 mg de tert-butoxycarbonyl-3 (méthoxy-4 phényl)-2 phényl-4 oxazolidine-1,3 carboxylate-5 (2R,4S,5R) d'acétoxy-4α benzoyloxy-2α cyclopentylcarbonyloxy-10β époxy-5β,20 hydroxy-1β méthylène-7β,8β oxo-9 nor-19 taxène-11 yle-13α, on obtient 100 mg de tert-butoxycarbonylamino-3 hydroxy-2 phényl-3 propionate-(2R,3S) d'acétoxy-4a benzoyloxy-2α cyclopentylcarbonyloxy-10β époxy-5β,20 hydroxy-1β méthylène-7β,8β oxo-9 nor-19 taxène-11 yle-13α sous forme d'une meringue blanche dont les caractéristiques sont les suivantes :
- pouvoir rotatoire : [α]^{D}₂₀ = - 40 (c = 0,5 ; méthanol)
- spectre de R.M.N. ¹H (300 MHz ; CDCI₃ ; déplacements chimiques δ en ppm ; constantes de couplage J en Hz) : 1,26 (s, 3H : -CH₃ en 16 ou en 17) ; 1,29 (s, 3H : -CH₃ en 16 ou en 17) ; 1,29 (s, 9H : -C(CH₃)₃) ; 1,40 (mt, 1H : -H en 7) ; de 1,50 à 1,80 [(mt, 4H : -C₅H₉ (-CH₂ en 3 et -CH₂ en 4) et 1H -CH₂- en 19)]; 1,86 (s, 4H : -CH₃ et -OH en 1) ; de 1,85 à 2,05 [(mt, 4H : -C₅H₉ (-CH₂ en 2 et -CH₂ en 5)] ; 2,11 et 2,47 (respectivement d large et dt, J = 16 et J = 16 et 4,5, 1H chacun : -CH₂- en 6) ; 2,22 et 2,38 (respectivement dd et mt, J = 15,5 et 9, 1H chacun : -CH₂- en 14) ; 2,25 (mt, 1H : -CH₂- en19) ; 2,39 (s, 3H : -COCH₃) ; 2,90 (mt, 1H: -C₅H₉ (-CH< en 1)]; 3,26 (mt, 1H : -OH en 2') ; 4,03 et 4,31 (2 d, J = 9, 1H chacun : -CH₂- en 20) ; 4,11 (d, J = 7,5, 1H : -H en 3) ; 4,62 (mt, 1H : -H en 2') ; 4,74 (d, J = 4,5, 1H : -H en 5) ; 5,28 (d large, J = 10, 1H : -H en 3') ; 5,35 (d, J = 10, 1H : -CONH-) ; 5,68 (d, J = 7,5, 1H : -H en 2) 6,27 (t large, J = 9, 1H : -H en 13) ; 6,32 (s, 1H : -H en 10) ; de 7,25 à 7,45 (mt, 5H : -C₆H₅ en 3') ; 7,51 [(t, J = 7,5, 2H : -OCOC₆H₅ (-H en 3 et H en 5)] ; 7,60 [(t, J = 7,5, 1H : -OCOC₆H₅ (H en 4)] ; 8,15 (d, J = 7,5, 2H : -OCOC₆H₅ (-H en 2 et H en 6)].

### EXEMPLE 4

En opérant comme dans l'exemple 1, mais à partir de 270 mg de tert-butoxycarbonyl-3 (méthoxy-4 phényl)-2 phényl-4 oxazolidine-1,3 carboxylate-5 (2R,4S,5R) d'acétoxy-4α benzoyloxy-2α dihydroxy-1β,10β époxy-5β,20 méthylène-7β,8β oxo-9 nor-19 taxène-11 yle-13α et de 0,028 cm3 d'acide cyclopropanecarboxylique, on obtient 110 mg de tert-butoxycarbonyl-3 (méthoxy-4 phényl)-2 phényl-4 oxazolidine-1,3 carboxylate-5 (2R,4S,5R) d'acétoxy-4α benzoyloxy-2α cyclopropylcarbonyloxy-10β époxy-5β,20 hydroxy-1β méthylène-7β,8β oxo-9 nor-19 taxène-11 yle-13α sous forme d'une meringue blanche.

En opérant comme dans l'exemple 1, mais à partir de 110 mg de tert-butoxycarbonyl-3 (méthoxy-4 phényl)-2 phényl-4 oxazolidine-1,3 carboxylate-5 (2R,4S,5R) d'acétoxy-4α benzoyloxy-2α cyclopropylcarbonyloxy-10β époxy-5β,20 hydroxy-1β méthylène-7β,8β oxo-9 nor-19 taxène-11 yle-13α, on obtient 62 mg de tert-butoxycarbonylamino-3 hydroxy-2 phényl-3 propionate-(2R,3S) d'acétoxy-4α benzoyloxy-2α cyclopropylcarbonyloxy-10β époxy-5β,20 hydroxy-1β méthylène-7β,8β oxo-9 nor-19 taxène-11 yle-13α sous forme d'une meringue blanche dont les caractéristiques sont les suivantes :
- pouvoir rotatoire : [α]^{D}₂₀ = - 35 (c = 0,5 ; méthanol)
- spectre de R.M.N. ¹H (400 MHz ; CDCI₃ ; déplacements chimiques δ en ppm ; constantes de couplage J en Hz) : 0,98 et 1,13 (2 mt, 2H chacun : -C₃H₅ (-CH₂- en 2 et -CH₂- en 3) ; 1,27 (s, 3H : -CH₃ en 16 ou en 17) ; 1,30 (s, 12H : -CH₃ en 16 ou en 17 et -C(CH₃)₃) ; 1,38 (mt, 1H : -H en 7) ; 1,67 et 2,26 (respectivement dd et mt, J = 6,5 et 5, 1H chacun : -CH₂- en 19) ; 1,74 (mt, 1H : -C₃H₅ (-CH< en 1) ; 1,86 (s, 4H : -OH en 1 et -CH₃) ; 2,12 et 2,44 (respectivement d large et dt, J = 16 et J = 16 et 4,5, 1H chacun : -CH₂- en 6) ; 2,25 et 2,38 (respectivement dd et mt, J = 16 et 9, 1H chacun : -CH₂- en 14) ; 2,38 (s, 3H : -COCH₃) ; 3,27 (mt, 1H : -OH en 2') ; 4,03 et 4,32 (2 d, J = 9, 1H chacun : -CH₂- en 20) ; 4,10 (d, J = 7,5, 1H : -H en 3) ; 4,62 (mt, 1H: -H en 2') ; 4,72 (d, J = 4,5, 1H: -H en 5) ; 5,29 (d large, J = 10, 1H : -H en 3') ; 5,36 (d, J = 10, 1H : -CONH-) ; 5,67 (d, J = 7,5, 1H : -H en 2) 6,28 (t large, J = 9, 1H : -H en 13) ; 6,34 (s, 1H : -H en 10) ; de 7,25 à 7,45 (mt, 5H :-C₆H₅ en 3') ; 7,51 [(t, J = 7,5, 2H : -OCOC₆H₅ (-H en 3 et H en 5)] ; 7,61 [(t, J = 7,5, 1H : -OCOC₆H₅ (-H en 4)] ; 8,15 [(d, J = 7,5, 2H : -OCOC₆H₅ (-H en 2 et H en 6)].

### EXEMPLE 5

En opérant comme dans l'exemple 1, mais à partir de 280 mg de tert-butoxycarbonyl-3 (méthoxy-4 phényl)-2 phényl-4 oxazolidine-1,3 carboxylate-5 (2R,4S,5R) d'acétoxy-4α benzoyloxy-2α dihydroxy-1β,10β époxy-5β,20 méthylène-7β,8β oxo-9 nor-19 taxène-11 yle-13α et de 41,5 mg d'acide furanne-2 carboxylique, on obtient 201 mg de tert-butoxycarbonyl-3 (méthoxy-4 phényl)-2 phényl-4 oxazolidine-1,3 carboxylate-5 (2R,4S,5R) d'acétoxy-4α benzoyloxy-2α époxy-5β,20 (furyl-2 carbonyl)oxy-10β hydroxy-1β méthylène-7β,8β oxo-9 nor-19 taxène-11 yle-13a sous forme d'une meringue blanche.

En opérant comme dans l'exemple 1, mais à partir de 197 mg de tert-butoxycarbonyl-3 (méthoxy-4 phényl)-2 phényl-4 oxazolidine-1,3 carboxylate-5 (2R,4S,5R) d'acétoxy-4α benzoyloxy-2α époxy-5β,20 (furyl-2 carbonyl)oxy-10β hydroxy-1β méthylène-7β,8β oxo-9 nor-19 taxène-11 yle-13α, on obtient 137 mg de tert-butoxycarbonylamino-3 hydroxy-2 phényl-3 propionate-(2R,3S) d'acétoxy-4α benzoyloxy-2α époxy-5β,20 (furyl-2 carbonyl)oxy-10β hydroxy-1β méthylène-7β,8β oxo-9 nor-19 taxène-11 yle-13α sous forme d'une meringue blanche dont les caractéristiques sont les suivantes :
- pouvoir rotatoire : [α]^{D}₂₀ = - 19 (c = 0,5 ; méthanol)
- spectre de R.M.N. ¹H (400 MHz ; CDCI₃ ; déplacements chimiques δ en ppm ; constantes de couplage J en Hz) : 1,28 (s, 9H : -C(CH₃)₃) ; 1,32 (s, 3H : -CH₃ en 16 ou en17) ; 1,38 (s, 3H : -CH₃ en 16 ou en17) ; 1,43 (mt, 1H : -H en 7) ; 1,70 et 2,28 (respectivement dd et mt, J = 7 et 5, 1H chacun : -CH₂- en 19) ; 1,89 (s, 1H : -OH en 1) ; 1,91 (s, 3H : -CH₃) ; 2,12 et 2,49 (respectivement d large et d, J = 16 et J = 16 et 4,5, 1H chacun : -CH₂- en 6) ; 2,27 et 2,40 (respectivement dd et mt, J = 16 et 9, 1H chacun : -CH₂- en 14) ; 2,40 (s, 3H : -COCH₃) ; 3,26 (mt, 1H : -OH en 2') ; 4,05 et 4,33 (2 d, J = 9, 1H chacun : -CH₂- en 20) ; 4,10 (d, J = 7,5, 1H : -H 3) ; 4,63 (mt, 1H : -H en 2') ; 4,72 (d, J = 4,5, 1H : -H en 5) ; 5,29 (d large, J = 10, 1H : -H en 3') ; 5,36 (d, J = 10, 1H : -CONH-) ; 5,71 (d, J = 7,5, 1H : -H en 2) 6,29 (t large, J = 9, 1H: -H en 13) ; 6,53 (s, 1H : -H en10) ; 6,56 [(dd, J = 5 et 1,5, 1H : -C₄H₃O (-H en 4)] ; 7,26 [(d, J = 4, 1H : -C₄H₃O (-H en 3)] ; de 7,25 à 7,45 (mt, 5H : -C₆H₅ en 3') ; 7,51 (t, J = 7,5, 2H : -OCOC₆H₅ (-H en 3 et H en 5)] ; 7,61 (t, J = 7,5, 1H : -OCOC₆H₅ (-H en 4)] ; 7,64 (s large, 1H : -C₄H₃O (-H en 5)] ; 8,15 (d, J = 7,5, 2H: -OCOC₆H₅ (-H en 2 et H en 6)].

### EXEMPLE 6

En opérant comme dans l'exemple 1, mais à partir de 280 mg de tert-butoxycarbonyl-3 (méthoxy-4 phényl)-2 phényl-4 oxazolidine-1,3 carboxylate-5 (2R,4S,5R) d'acétoxy-4α benzoyloxy-2α dihydroxy-1β,10β époxy-5β,20 méthylène-7β,8β oxo-9 nor-19 taxène-11 yle-13α et de 45 mg d'acide benzoïque, on obtient 190 mg de tert-butoxycarbonyl-3 (méthoxy-4 phényl)-2 phényl-4 oxazolidine-1,3 carboxylate-5 (2R,4S,5R) d'acétoxy-4α dibenzoyloxy-2α,10β époxy-5β,20 hydroxy-1β méthylène-7β,8β oxo-9 nor-19 taxène-11 yle-13α sous forme d'une meringue blanche.

En opérant comme dans l'exemple 1, mais à partir de 190 mg de tertbutoxycarbonyl-3 (méthoxy-4 phényl)-2 phényl-4 oxazolidine-1,3 carboxylate-5 (2R,4S,5R) d'acétoxy-4α dibenzoyloxy-2α,10β époxy-5β,20 hydroxy-1β méthylène-7β,8β oxo-9 nor-19 taxène-11 yle-13α, on obtient 120 mg de tert-butoxycarbonylamino-3 hydroxy-2 phényl-3 propionate-(2R,3S) d'acétoxy-4α dibenzoyloxy-2α,10β époxy-5β,20 hydroxy-1β méthylène-7β,8β oxo-9 nor-19 taxène-11 yle-13α sous forme d'une meringue blanche dont les caractéristiques sont les suivantes :
- pouvoir rotatoire : [α]^{D}₂₀ = - 28 (c = 0,5 ; méthanol)
- spectre de R.M.N. ¹H (400 MHz ; CDCI₃ ; déplacements chimiques δ en ppm ; constantes de couplage J en Hz) : 1,29 (s, 9H : -C(CH₃)₃) ; 1,34 (s, 3H : -CH₃ en 16 ou en17) ; 1,42 (s, 3H : -CH₃ en 16 ou en 17) ; 1,42 (mt, 1H : -H en 7) ; 1,69 et 2,27 (respectivement dd et mt, J = 7 et 5, 1H chacun : -CH₂- en 19) ; 1,91 (s, 1H : -OH en 1) ; 1,92 (s, 3H : -CH₃) ; 2,13 et 2,50 (respectivement d large et dt, J = 16 et J = 16 et 4,5, 1H chacun : -CH₂- en 6) ; 2,26 et 2,41 (respectivement dd et mt, J = 16 et 9, 1H chacun : -CH₂- en 14) ; 2,41 (s, 3H : -COCH₃) ; 3,26 (mt, 1H : -OH en 2') ; 4,07 et 4,34 (2 d, J = 9, 1H chacun : -CH₂- en 20) ; 4,18 (d, J = 7,5, 1H : -H en 3) ; 4,63 (mt, 1H : -H en 2') ; 4,75 (d, J = 4,5, 1H : -H en 5) ; 5,29 (d large, J = 10, 1H : -H en 3') ; 5,37 (d, J = 10, 1H : -CONH-) ; 5,73 (d, J = 7,5, 1H : -H en 2) 6,29 (t large, J = 9, 1H : -H en 13) ; 6,60 (s, 1H : -H en 10); de 7,25 à 7,45 (mt, 5H : -C₆H₅ en 3') ; 7,49 et 7,51 [(2 t, J = 7,5, 2H chacun : -OCOC₆H₅ (-H en 3 et H en 5)] ; de 7,55 à 7,65 [(mt, 2H : -OCOC₆H₅ (-H en 4)] ; 8,09 et 8,17 [(2 d, J = 7,5, 2H chacun: -OCOC₆H₅ (-H en 2 et H en 6)].

### EXEMPLE 7

En opérant comme dans l'exemple 1, mais à partir de 300 mg de tert-butoxycarbonyl-3 (méthoxy-4 phényl)-2 phényl-4 oxazolidine-1,3 carboxylate-5 (2R,4S,5R) d'acétoxy-4α benzoyloxy-2α dihydroxy-1β,10β époxy-5β,20 méthylène-7β,8β oxo-9 nor-19 taxène-11 yle-13α et de 0,686 cm3 d'anhydride méthyl-3 propén-2 oïque, on obtient 237 mg de tert-butoxycarbonyl-3 (méthoxy-4 phényl)-2 phényl-4 oxazolidine-1,3 carboxylate-5 (2R,4S,5R) d'acétoxy-4α benzoyloxy-2α époxy-5β,20 hydroxy-1β (méthyl-3 propén-2 oyl)oxy-10β méthylène-7β,8β oxo-9 nor-19 taxène-11 yle-13α sous forme d'une meringue blanche.

En opérant comme dans l'exemple 1, mais à partir de 270 mg de tert-butoxycarbonyl-3 (méthoxy-4 phényl)-2 phényl-4 oxazolidine-1,3 carboxylate-5 (2R,4S,5R) d'acétoxy-4α benzoyloxy-2α époxy-5β,20 hydroxy-1β (méthyl-3 propén-2 oyl)oxy-10β méthylène-7β,8β oxo-9 nor-19 taxène-11 yle-13α, on obtient 192 mg de tert-butoxycarbonylamino-3 hydroxy-2 phényl-3 propionate-(2R,3S) d'acétoxy-4α benzoyloxy-2α époxy-5β,20 hydroxy-1β (méthyl-3 propén-2 oyl)oxy-10β méthylène-7β,8β oxo-9 nor-19 taxène-11 yle-13α sous forme d'une meringue blanche dont les caractéristiques sont les suivantes :
- pouvoir rotatoire : [α]^{D}₂₀ = -34 (c = 0,5 ; méthanol)
- spectre de R.M.N. 1H (400 MHz ; CDCI₃ ; déplacements chimiques δ en ppm ; constantes de couplage J en Hz) : 1,27 (s, 12H : -C(CH₃)₃ et -CH₃ en 16 ou en 17) ; 1,30 (s, 3H : -CH₃ en 16 ou en 17) ; 1,39 (mt, 1H : -H en 7) ; 1,67 et 2,26 (respectivement dd et mt, J = 6,5 et 5,5, 1H chacun : -CH₂ en 19) ; 1,86 (s, 4H : -OH en 1 et -CH₃) ; 1,93 (dd, J = 7,5 et 1,5, 3H : -CH₃) ; 2,11 et 2,47 (respectivement d large et dt, J = 16 et J = 16 et 4,5, 1H chacun : -CH₂- en 6) ; 2,23 et 2,39 (respectivement dd et mt, J = 16 et 9, 1H chacun : -CH₂- en 14) ; 2,38 (s, 3H : -COCH₃) ; 3,25 (mt, 1H : -OH en 2') ; 4,04 et 4,30 (2 d, J = 9, 1H chacun : -CH₂- en 20) ; 4,12 (d, J = 7,5, 1H : -H en 3) ; 4,62 (mt, 1H : -H en 2') ; 4,73 (d, J = 4,5, 1H : -H en 5) ; 5,29 (d large, J = 10, 1H : -H en 3') ; 5,36 (d, J = 10, 1H : -CONH-) ; 5,68 (d, J = 7,5, 1H : -H en 2) 5,98 [(dd, J = 16 et 1,5, 1H : -OCOCH=CH-CH₃)] ; 6,27 (t large, J = 9, 1H : -H en13) ; 6,40 (s, 1H : -H en 10) ; 7,07 [(dt, J = 16 et 7,5, 1H : -OCOCH=CH-CH₃)] ; de 7,25 à 7,50 (mt, 5H : -C₆H₅ en 3') ; 7,51 [(t, J = 7,5, 2H : -OCOC₆H₅ (-H en 3 et H en 5)] ; 7,61 [(t, J = 7,5, 1H : -OCOC₆H₅ (-H en 4)] ; 8,15 [(d, J = 7,5, 2H : -OCOC₆H₅ (-H en 2 et H en 6)].

### EXEMPLE 8

En opérant comme dans l'exemple 1, mais à partir de 220 mg de tert-butoxycarbonyl-3 (méthoxy-4 phényl)-2 phényl-4 oxazolidine-1,3 carboxylate-5 (2R,4S,5R) d'acétoxy-4α benzoyloxy-2α dihydroxy-1β,10β époxy-5β,20 méthylène-7β,8β oxo-9 nor-19 taxène-11 yle-13α et de 28 mg d'acide chloroacétique, on obtient 100 mg de tert-butoxycarbonyl-3 (méthoxy-4 phényl)-2 phényl-4 oxazolidine-1,3 carboxylate-5 (2R,4S,5R) d'acétoxy-4α benzoyloxy-2α chloroacétoxy-10β époxy-5β,20 hydroxy-1β méthylène-7β,8β oxo-9 nor-19 taxène-11 yle-13α sous forme d'une meringue blanche.

En opérant comme dans l'exemple 1, mais à partir de 155 mg de tert-butoxycarbonyl-3 (méthoxy-4 phényl)-2 phényl-4 oxazolidine-1,3 carboxylate-5 (2R,4S,5R) d'acétoxy-4α benzoyloxy-2α chloroacétoxy-10β époxy-5β,20 hydroxy-1β méthylène-7β,8β oxo-9 nor-19 taxène-11 yle-13α, on obtient 64 mg de tert-butoxycarbonylamino-3 hydroxy-2 phényl-3 propionate-(2R,3S) d'acétoxy-4α benzoyloxy-2α chloroacétoxy-10β époxy-5β,20 hydroxy-1β méthylène-7β,8β oxo-9 nor-19 taxène-11 yle-13α sous forme d'une meringue blanche dont les caractéristiques sont les suivantes :
- pouvoir rotatoire : [α]^{D}₂₀ = -39 (c = 0,5 ; méthanol)
- spectre de R.M.N. ¹H (400 MHz ; CDCI₃ ; déplacements chimiques δ en ppm ; constantes de couplage J en Hz) : 1,27 (s, 3H : -CH₃ en 16 ou en 17) ; 1,29 (s, 12H : -CH₃ en 16 ou en 17 et -C(CH₃)₃) ; 1,39 (mt, 1H : -H en 7) ; 1,71 et 2,26 (respectivement dd et mt, J = 7 et 5,5, 1H chacun : -CH₂- en 19) ; 1,87 (s, 4H : -OH en 1 et -CH₃) ; 2,12 et 2,47 (respectivement d large et dt, J = 16 et J = 16 et 4,5, 1H chacun : -CH₂- en 6) ; 2,27 et 2,38 (respectivement dd et mt, J = 16 et 9, 1H chacun: -CH₂- en 14) ; 2,40 (s, 3H : -COCH₃) ; 3,27 (mt, 1H : -OH en 2') ; 4,03 et 4,32 (2 d, J = 9, 1H chacun : -CH₂- en 20) ; 4,07 (d, J = 7,5, 1H : -H en 3) ; 4,26 (AB limite, J = 16, 2H : -OCOCH₂CI) ; 4,62 (mt, 1H : -H en 2') ; 4,74 (d, J = 4,5, 1H : -H en 5) ; 5,28 (d large, J = 10 Hz, 1H : -H en 3') ; 5,35 (d, J = 10, 1H : -CONH-) ; 5,68 (d, J = 7,5, 1H: -H 2) ; 6,28 (t large, J = 9 Hz, 1H : -H en 13) ; 6,38 (s, 1H : -H en 10) ; de 7,25 à 7,45 (mt, 5H : -C₆H₅ en 3') ; 7,51 [(t, J = 7,5, 2H : -OCOC₆H₅ (-H en 3 et H en 5)] ; 7,61 [(t, J = 7,5, 1H : -OCOC₆H₅ (-H en 4)] ; 8,16 (d, J = 7,5, 2H : -OCOC₆H₅ (-H en 2 et H en 6)].

### EXEMPLE 9

En opérant comme dans l'exemple 7, mais à partir de 300 mg de tert-butoxycarbonyl-3 (méthoxy-4 phényl)-2 phényl-4 oxazolidine-1,3 carboxylate-5 (2R,4S,5R) d'acétoxy-4α benzoyloxy-2α dihydroxy-1β,10β époxy-5β,20 méthylène-7β,8β oxo-9 nor-19 taxène-11 yle-13α et de 52,4 mg d'acide éthoxycarbonylacétique, on obtient 180 mg de tert-butoxycarbonyl-3 (méthoxy-4 phényl)-2 phényl-4 oxazolidine-1,3 carboxylate-5 (2R,4S,5R) d'acétoxy-4α benzoyloxy-2α époxy-5β,20 éthoxycarbonylacétoxy-10β hydroxy-1β méthylène-7β,8β oxo-9 nor-19 taxène-11 yle-13α sous forme d'une meringue blanche.

En opérant comme dans l'exemple 7, mais à partir de 190 mg de tert-butoxycarbonyl-3 (méthoxy-4 phényl)-2 phényl-4 oxazolidine-1,3 carboxylate-5 (2R,4S,5R) d'acétoxy-4α benzoyloxy-2α époxy-5β,20 éthoxycarbonylacétoxy-10β hydroxy-1β méthylène-7β,8β oxo-9 nor-19 taxène-11 yle-13α, on obtient 73 mg de tert-butoxycarbonylamino-3 hydroxy-2 phényl-3 propionate-(2R,3S) d'acétoxy-4α benzoyloxy-2α époxy-5β,20 éthoxycarbonylacétoxy-10β hydroxy-1β méthylène-7β,8β oxo-9 nor-19 taxène-11 yle-13α sous forme d'une meringue blanche dont les caractéristiques sont les suivantes :
- pouvoir rotatoire : [α]^{D}₂₀ = -28 (c = 0,5 ; méthanol)
- spectre de R.M.N. ¹H (300 MHz ; CDCI₃ ; déplacements chimiques δ en ppm ; constantes de couplage J en Hz) :1,24 (s, 3H : -CH₃ en 16 ou en 17) ; 1,28 (s, 12H : -C(CH₃) ₃ et -CH₃ en 16 ou en 17) ; 1,32 (t, J = 7,5, 3H : -OCOCH₂COOCH₂CH₃) ; 1,41 (mt, 1H : -H en 7) ; 1,72 et 2,22 (respectivement dd et mt, J = 6,5 et 5, 1H chacun : -CH₂- en 19) ; 1,89 (s, 4H : -OH en 1 et -CH₃) ; 2,15 et 2,50 (respectivement d large et dt, J = 16 et J = 16 et 4,5, 1H chacun : -CH₂- en 6) ; 2,28 et 2,40 (respectivement dd et mt, J = 16 et 9, 1H chacun : -CH₂- en 14) ; 2,42 (s, 3H: -COCH₃) ; 3,32 (mt, 1H : -OH en 2') ; 3,58 (AB limite, 2H : -OCOCH₂COOCH₂CH₃) ; 4,08 et 4,35 (2 d, J = 9 Hz, 1H chacun : -CH₂- en 20) ; 4,10 (d, J = 7,5, 1H : -H en 3) ; 4,28 (q, J = 7,5, 2H : -OCOCH₂COOCH₂CH₃) ; 4,63 (mt, 1H : -H en 2') ; 4,75 (d, J = 4,5, 1H : -H en 5) ; 5,29 (d large, J = 10, 1H : -H en 3') ; 5,39 (d, J = 10, 1H : -CONH-) ; 5,69 (d, J = 7,5, 1H : -H en 2) ; 6,30 (t large, J = 9, 1H : -H en 13) ; 6,38 (s, 1H : -H en 10) ; de 7,25 à 7,50 (mt, 5H : -C₆H₅ en 3') ; 7,53 [(t, J = 7,5, 2H :-OCOC₆H₅ (-H en 3 et H en 5)] ; 7,62 [(t, J = 7,5, 1H : -OCOC₆H₅ (-H en 4)] ; 8,18 [(d, J = 7,5, 2H : -OCOC₆H₅(-H en 2 et H -en 6)].

### EXEMPLE 10

En opérant comme dans l'exemple 1, mais à partir de 300 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α dihydroxy-1β,10β époxy-5β,20 méthylène-7β,8β oxo-9 nor-19 taxène-11 yle-13α et de 584 mg d'anhydride acrylique, on obtient 160 mg de tert-butoxycarbonyl-3 (méthoxy-4 phényl)-2 phényl-4 oxazolidine-1,3 carboxylate-5-(2R,4S,5R) d'acétoxy-4α acryloyloxy-10β benzoyloxy-2α époxy-5β,20 hydroxy-1β méthylène-7β,8β oxo-9 nor-19 taxène-11 yle-13α sous forme d'une meringue blanche.

En opérant comme dans l'exemple 1, mais à partir de 196 mg de tert-butoxycarbonyl-3 (méthoxy-4 phényl)-2 phényl-4 oxazolidine-1,3 carboxylate-5-(2R,4S,5R) d'acétoxy-4α acryloyloxy-10β benzoyloxy-2α époxy-5β,20 hydroxy-1β méthylène-7β,8β oxo-9 nor-19 taxène-11 yle-13α, on obtient 113 mg de tert-butoxycarbonylamino-3 hydroxy-2 phényl-3 propionate-(2R,3S) d'acétoxy-4α acryloyloxy-10β benzoyloxy-2α époxy-5β,20 hydroxy-1β méthylène-7β,8β oxo-9 nor-19 taxène-11 yle-13α sous forme d'une meringue blanche dont les caractéristiques sont les suivantes :
- pouvoir rotatoire : [α]^{D}₂₀ = -39 (c = 0,5 ; méthanol)
- spectre de R.M.N. ¹H (400 MHz ; CDCI₃ ; déplacements chimiques δ en ppm ; constantes de couplage J en Hz) : 1,27 [s, 12H : -C(CH₃) 3 et CH₃ en 16 ou en 17] ; 1,30 (s, 3H : -CH₃ en 16 ou en 17) ; 1,39 (mt, 1H : -H en 7) ; 1,68 et 2,26 (respectivement dd et mt, J = 6,5 et 5, 1H chacun : -CH₂- en 19) ; 1,86 (s, 1H : -OH 1) ; 1,88 (s, 3H : -CH₃) ; 2,13 et 2,49 (respectivement d large et dt, J = 15 et J = 15 et 4, 1H chacun : -CH₂- en 6) ; 2,25 et 2,39 (2 mts, 1H chacun : -CH₂- en 14) ; 2,38 (s, 3H : -COCH₃) ; 3,27 (mt, 1H : -OH en 2') ; 4,06 et 4,34 (2 d, J = 9, 1H chacun : -CH₂- en 20) ; 4,13 (d, J = 7, 1H : -H en 3) ; 4,63 (mt, 1H : -H en 2') ; 4,75 (d, J = 4, 1H : -H en 5) ; 5,29 (d large, J = 10, 1H : -H en 3') ; 5,35 (d, J = 10, 1H : -CONH-) ; 5,69 (d, J = 7, 1H : -H en 2) 5,95 et 6,53 (2 dd, respectivement J = 10 et 1,5 et J = 16 et 1,5, 1H chacun : -OCOCH=CH₂); 6,27 (dd, J = 16 et 10, 1H : -OCOCH=CH₂) ; 6,29 (mt, 1H : -H en 13) ; 6,42 (s, 1H : -H en 10) ; de 7,25 à 7,45 (mt, 5H : -C₆H₅ en 3') ; 7,53 [(t, J = 7,5, 2H : -OCOC₆H₅(-H en 3 et H en 5)] ; 7,63 [(t, J = 7,5, 1H : -OCOC₆H₅(-H en 4)] ; 8,17 (d, J = 7,5, 2H : -OCOC₆H₅(-H en 2 et H- en 6)].

### EXEMPLE 11

En opérant comme dans l'exemple 1, mais à partir de 250 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α dihydroxy-1β,10β époxy-5β,20 méthylène-7β,8β oxo-9 nor-19 taxène-11 yle-13α et de 41 mg d'acide pyridine-3 carboxylique, on obtient 269 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5(3,20 hydroxy-1β méthylène-7β,8β oxo-9 (pyridyl-3 carbonyl)oxy-10β nor-19 taxène-11 yle-13α sous forme d'une meringue blanche.

En opérant comme dans l'exemple 1, mais à partir de 264 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β méthylène-7β,8β oxo-9 (pyridyl-3 carbonyl)oxy-10β nor-19 taxène-11 yle-13α, on obtient 169 mg de tert-butoxycarbonylamino-3 hydroxy-2 phényl-3 propionate-(2R,3S) d'acétoxy-4α benzoyloxy-2α époxy-5β,20 hydroxy-1β méthylène-7β,8β oxo-9 (pyridyl-3 carbonyl)oxy-10β nor-19 taxène-11 yle-13α sous forme d'une meringue blanche dont les caractéristiques sont les suivantes :
- pouvoir rotatoire : [α]^{D}₂₀ = -25 (c = 0,5 ; méthanol)
- spectre de R.M.N. ¹H (400 MHz ; CDCl₃ ; déplacements chimiques δ en ppm ; constantes de couplage J en Hz) : 1,29 [(s, 9H : -C(CH₃)₃] ; 1,34 (s, 3H : -CH₃ en 16 ou en17) ; 1,41 (s, 3H : -CH₃ en 16 ou en 17) ; 1,45 (mt, 1H : -H en 7) ; 1,74 et 2,29 (respectivement dd et mt, J = 6,5 et 5,5, 1H chacun : -CH₂- en 19) ; 1,94 (s, 3H : -CH₃) ; 2,14 et 2,52 (respectivement d large et dt, J = 16 et J = 16 et 4, 1H chacun : -CH₂- en 6) ; 2,29 et 2,43 (2 mts, 1H chacun : -CH₂- en 14) ; 2,43 (s, 3H : -COCH₃) ; 3,31 (mt, 1H : -OH en 2') ; 4,07 et 4,35 (2 d, J = 9, 1H chacun : -CH₂- en 20) ; 4,17 (d, J = 7,5, 1H : -H en 3) ; 4,64 (mt, 1H : -H en 2') ; 4,77 (d, J = 4, 1H : -H en 5) ; 5,30 (d large, J = 10, 1H : -H en 3') ; 5,37 (d, J = 10, 1H : -CONH-) ; 5,74 (d, J = 7,5, 1H : -H en 2) 6,32 (t large, J = 8,5, 1H : -H en 13) ; 6,63 (s, 1H : -H en 10); de 7,25 à 7,45 (mt, 5H : -C₆H₅ en 3') ; 7,45 [(dd, J = 8 et 5,5, 1H : -OCOC₅H₄N(-H en 5)] ; 7,53 ((t, J = 7,5, 2H : -OCOC₆H₅(-H en 3 et H en 5)] ; 7,63 [(t, J = 7,5, 1H : -OCOC₆H₅(-H en 4)] ; 8,18 [(d, J = 7,5, 2H : -OCOC₆H₅(-H en 2 et -H en 6)] ; 8,36 [(dt, J = 8 et 1,5, 1H : -OCOC₅H₄N(-H en 4)] ; 8,84 (dd, J = 5,5 et 1,5, 1H : -OCOC₅H₄N(-H en 6)] ; 9,29 (d, J = 1,5, 1H : -OCOC₅H₄N(-H en 2)].

### EXEMPLE 12

En opérant comme dans l'exemple 1, mais à partir de 250 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α dihydroxy-1β,10β époxy-5β,20 méthylène-7β,8β oxo-9 nor-19 taxène-11 yle-13α et de 42 mg d'acide thiophène-3 carboxylique, on obtient 180 mg de tert-butoxycarbonyl-3 (méthoxy-4 phényl)-2 phényl-4 oxazolidine-1,3 carboxylate-5 (2R,4S,5R) d'acétoxy-4α benzoyloxy-2α époxy-5(3,20 hydroxy-1β méthylène-7β,8β oxo-9 (thénoyl-3)oxy-10β nor-19 taxène-11 yle-13α sous forme d'une meringue blanche.

En opérant comme dans l'exemple 1, mais à partir de 175 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β méthylène-7β,8β oxo-9 (thénoyl-3)oxy-10β nor-19 taxène-11 yle-13α, on obtient 102 mg de tert-butoxycarbonylamino-3 hydroxy-2 phényl-3 propionate-(2R,3S) d'acétoxy-4α benzoyloxy-2α époxy-5β,20 hydroxy-1β méthylène-7β,8β oxo-9 (thénoyl-3)oxy-10β nor-19 taxène-11 yle-13α sous forme d'une meringue blanche dont les caractéristiques sont les suivantes :
- pouvoir rotatoire : [α]^{D}₂₀ = - 16 (c = 0,5 ; méthanol)
- spectre de R.M.N. ¹H (400 MHz ; CDCl₃ ; déplacements chimiques δ en ppm ; constantes de couplage J en Hz) : 1,30 [s, 9H : -C(CH₃)₃] ; 1,33 (s, 3H : -CH₃ en 16 ou en 17) ; 1,40 (s, 3H : -CH₃ en 16 ou en 17) ; 1,44 (mt, 1H : -H en 7) ; 1,72 et 2,29 (2 dd, respectivement J = 6,5 et 5,5 et J = 10 et 6,5, 1H chacun : -CH₂- 19) ; 1,92 (s, 4H : -CH₃ et -OH en 1) ; 2,14 et 2,51 (respectivement d large et dt, J = 16 et J = 16 et 4, 1H chacun : -CH₂- en 6) ; 2,26 et 2,42 (2 mts, 1H chacun : -CH₂- en 14) ; 2,42 (s, 3H : -COCH₃) ; 3,27 (mt, 1H : -OH en 2') ; 4,06 et 4,32 (2 d, J = 9, 1H chacun : -CH₂- en 20) ; 4,17 (d, J = 7,5, 1H : -H en 3) ; 4,63 (mt, 1H : -H en 2') ; 4,76 (d, J = 4, 1H : -H en 5) ; 5,29 (d large, J = 10, 1H : -H en 3') ; 5,35 (d, J = 10, 1H : -CONH-) ; 5,72 (d, J = 7,5, 1H : -H en 2) 6,30 (t large, J = 8,5, 1H : -H en 13) ; 6,53 (s, 1H : -H en 10) ; de 7,25 à 7,45 [mt, 6H : -C₆H₅ en 3' et -OCOC₄H₃S(-H en 5)] ; 7,53 [(t, J = 7,5, 2H : -OCOC₆H₅(-H en 3 et H en 5)] ; 7,57 [d large, J = 5,5, 1H : -OCOC₄H₃S(-H en 4)] ; 7,62 [(t, J = 7,5, 1H : -OCOC₆H₅(-H en 4)] ; 8,17 [(d, J = 7,5, 2H : -OCOC₆H₅(-H en 2 et H- en 6)] ; 8,19 [mt, 1H : -OCOC₄H₃S(-H en 2)].

### EXEMPLE 13

En opérant comme dans l'exemple 1, mais à partir de 300 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α dihydroxy-1β,10β époxy-5β,20 méthylène-7β,8β oxo-9 nor-19 taxène-11 yle-13α et de 713 mg d'anhydride vinylacétique, on obtient 114 mg de tert-butoxycarbonyl-3 (méthoxy-4 phényl)-2 phényl-4 oxazolidine-1,3 carboxylate-5-(2R,4S,5R) d'acétoxy-4α allylcarbonyloxy-10β benzoyloxy-2α époxy-5β,20 hydroxy-1β méthylène-7β,8β oxo-9 nor-19 taxène-11 yle-13α sous forme d'une meringue blanche.

L'anhydride vinylacétique peut être préparée de la manière suivante :

A une solution de 3,42 g de N,N'-dicyclohexylcarbodiimide dans 20 cm3 de dichlorométhane, maintenue sous atmosphère d'argon et sous agitation, on ajoute, goutte à goutte et à une température voisine de 20°C, 2,8 cm3 d'acide vinylacétique. Le milieu réactionnel est maintenu sous agitation, à une température voisine de 20°C, pendant 3 jours puis filtré sur verre fritté garni de célite. Le verre fritté est lavé par 2 fois 10 cm3 de dichlorométhane, les filtrats sont réunis et concentrés à sec sous pression réduite (2,7 kPa) à 40°C. On obtient ainsi 2,96 g d'anhydride vinylacétique sous forme d'une huile jaune.

En opérant comme dans l'exemple 1, mais à partir de 140 mg de tert-butoxycarbonyl-3 (méthoxy-4 phényl)-2 phényl-4 oxazolidine-1,3 carboxylate-5-2R,4S,5R) d'acétoxy-4α allylcarbonyloxy-10β benzoyloxy-2α époxy-5β,20 hydroxy-1β méthylène-7β,8β oxo-9 nor-19 taxène-11 yle-13α, on obtient 80 mg de tert-butoxycarbonylamino-3 hydroxy-2 phényl-3 propionate-(2R,3S) d'acétoxy-4α allylcarbonyloxy-10β benzoyloxy-2α époxy-5β,20 hydroxy-1β méthylène-7β,8β oxo-9 nor-19 taxène-11 yle-13α sous forme d'une meringue blanche dont les caractéristiques sont les suivantes :
- pouvoir rotatoire : [α]^{D}₂₀ = -34 (c = 0,5 ; méthanol)
- spectre de R.M.N. ¹H (400 MHz ; CDCl₃ ; déplacements chimiques δ en ppm ; constantes de couplage J en Hz) : 1,26 (s, 3H : -CH₃ en 16 ou en17) ; 1,29 [s, 12H : -C(CH₃)₃ et -CH₃ en 16 ou en 17] ; 1,38 (mt, 1H : -H en 7) ; 1,69 et 2,26 (2 dd, respectivement J = 6,5 et 5,5 et J = 10 et 6,5, 1H chacun : -CH₂- en 19) ; 1,84 (s, 1H: -OH en1) ; 1,85 (s, 3H : -CH₃) ; 2,12 et 2,46 (respectivement d large et dt, J = 16 et J = 16 et 4, 1H chacun : -CH₂- en 6) ; 2,24 et 2,40 (2 mts, 1H chacun : -CH₂- en 14); 2,40 (s, 3H : -COCH₃) ; 3,27 (mt, 3H : -OH en 2' et OCOCH₂-CH=CH₂) ; 4,05 et 4,32 (2 d, J = 9, 1H chacun : -CH₂- en 20) ; 4,11 (d, J = 7,5, 1H : -H en 3) ; 4,63 (mt, 1H: -H en 2') ; 4,73 (d, J = 4, 1H : -H en 5) ; 5,24 et 5,26 (2dd, respectivement J = 8 et 2 et J = 18 et 2, 1H chacun : OCOCH₂-CH=CH₂) ; 5,29 (d large, J = 10, 1H : -H 3') ; 5,34 (d, J = 10, 1H : -CONH-) ; 5,69 (d, J = 7,5, 1H : -H en 2) 6,00 (mt, 1H : OCOCH₂-CH=CH₂) ; 6,28 (t large, J = 8,5, 1H : -H en 13) ; 6,34 (s, 1H : -H en 10) ; de 7,25 à 7,45 (mt, 5H : -C₆H₅ en 3') ; 7,53 [(t, J = 7,5, 2H : -OCOC₆H₅(-H en 3 et H en 5)] ; 7,62 [(t, J = 7,5, 1H : -OCOC₆H₅(-H en 4)] ; 8,15 (d, J = 7,5, 2H : -OCOC₆H₅(-H en 2 et H- en 6)].

### EXEMPLE 14

En opérant comme dans l'exemple 1, mais à partir de 300 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α dihydroxy-1β,10β époxy-5β,20 méthylène-7β,8β oxo-9 nor-19 taxène-11 yle-13α et de 45 mg d'acide furanne-3 carboxylique, on obtient 282 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 (furoyl-3)oxy-10β hydroxy-1β méthylène-7β,8β oxo-9 nor-19 taxène-11 yle-13α sous forme d'une meringue blanche.

En opérant comme dans l'exemple 1, mais à partir de 282 mg de tert-butoxycarbonyl-3 (méthoxy-4 phényl)-2 phényl-4 oxazolidine-1,3 carboxylate-5 (2R,4S,5R) d'acétoxy-4α benzoyloxy-2α époxy-5β,20 (furoyl-3)oxy-10β hydroxy-1β méthylène-7β,8β oxo-9 nor-19 taxène-11 yle-13α, on obtient 143 mg de tert-butoxycarbonylamino-3 hydroxy-2 phényl-3 propionate-(2R,3S) d'acétoxy-4α benzoyloxy-2α époxy-5β,20 (furoyl-3)oxy-10β hydroxy-1β méthylène-7β,8β oxo-9 nor-19 taxène-11 yle-13α sous forme d'une meringue blanche dont les caractéristiques sont les suivantes :
- pouvoir rotatoire : [α]^{D}₂₀ = - 26 (c = 0,5 ; méthanol)
- spectre de R.M.N. ¹H (400 MHz ; CDCI₃ ; déplacements chimiques δ en ppm ; constantes de couplage J en Hz) : 1,29 [mt, 12H : -C(CH₃)₃ et -CH₃ en 16 ou en 17] ; 1,35 (s, 3H : -CH₃ en 16 ou en 17) ; 1,43 (mt, 1H : -H en 7) ; 1,70 et 2,27 (2 dd, respectivement J = 6,5 et 5,5 et J = 10 et 5,5, 1H chacun : -CH₂- 19) ; 1,87 (s, 1H : -OH en 1) ; 1,92 (s, 3H : -CH₃) ; 2,13 et 2,50 (respectivement d large et dt, J = 16 et J = 16 et 4, 1H chacun : -CH₂- en 6) ; 2,27 et 2,40 (2 mts, 1H chacun : -CH₂- en 14); 2,40 (s, 3H : -COCH₃) ; 3,27 (mt, 1H : -OH en 2') ; 4,05 et 4,33 (2 d, J = 9, 1H chacun : -CH₂- en 20) ; 4,15 (d, J = 7,5, 1H : -H en 3) ; 4,63 (mt, 1H : -H en 2') ; 4,76 (d, J = 4, 1H : -H en 5) ; 5,29 (d large, J = 10, 1H : -H en 3') ; 5,36 (d, J = 10, 1H : -CONH-) ; 5,72 (d, J = 7,5, 1H : -H en 2) ; 6,30 (t large, J = 8,5, 1H : -H en 13) ; 6,52 (s, 1H : -H en 10) ; 6,79 (d, J = 1,5, 1H : -OCOC₄H₃O(-H en 4)] ; de 7,25 à 7,45 (mt, 5H : -C₆H₅ en 3') ; 7,48 (t large, J = 1,5, 1H : -OCOC₄H₃O(-H en 5)] ; 7,53 [(t, J = 7,5, 2H : -OCOC6H₅(-H en 3 et H en 5)] ; 7,63 [(t, J = 7,5, 1H : -OCOC6H₅(-H en 4)] ; 8,09 [s large,1H : -OCOC₄H₃O(-H en 2)] ; 8,17 [(d, J = 7,5, 2H : -OCOC₆H₅(-H en 2 et H- en 6)].

### EXEMPLE 15

En opérant comme dans l'exemple 1, mais à partir de 300 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α dihydroxy-1β,10β époxy-5β,20 méthylène-7β,8β oxo-9 nor-19 taxène-11 yle-13α et de 50 mg d'acide pyridine-4 carboxylique, on obtient 296 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β méthylène-7β,8β oxo-9 (pyridyl-4 carbonyl)oxy-10β nor-19 taxène-11 yle-13α sous forme d'une meringue blanche.

En opérant comme dans l'exemple 1, mais à partir de 296 mg de tert-butoxycarbonyl-3 (méthoxy-4 phényl)-2 phényl-4 oxazolidine-1,3 carboxylate-5 (2R,4S,5R) d'acétoxy-4α benzoyloxy-2α époxy-5β,20 hydroxy-1β méthylène-7β,8β oxo-9 (pyridyl-4 carbonyl)oxy-10β nor-19 taxène-11 yle-13α, on obtient 159 mg de tert-butoxycarbonylamino-3 hydroxy-2 phényl-3 propionate-(2R,3S) d'acétoxy-4α benzoyloxy-2α époxy-5β,20 hydroxy-1β méthylène-7β,8β oxo-9 (pyridyl-3 carbonyl)oxy-10β nor-19 taxène-11 yle-13α sous forme d'une meringue blanche dont les caractéristiques sont les suivantes :
- pouvoir rotatoire : [α]^{D}₂₀ = -23 (c = 0,5 ; méthanol)
- spectre de R.M.N. ¹H (300 MHz ; CDCI₃ ; déplacements chimiques δ en ppm ; constantes de couplage J en Hz) : 1,27 [(s, 9H : -C(CH₃)₃] ; 1,32 (s, 3H : -CH₃ en 16 ou en17) ; 1,39 (s, 3H : -CH₃ en 16 ou en 17) ; 1,42 (mt, 1H : -H en 7) ; 1,70 et 2,27 (respectivement dd et mt, J = 6 et 5,5, 1H chacun : -CH₂-en 19) ; 1,90 (s, 3H : -CH₃) ; 1,91 (s, 1H : -OH en 1) ; 2,13 et 2,50 (respectivement d large et dt, J = 16 et J = 16 et 4, 1H chacun : -CH₂- en 6) ; 2,27 et 2,40 (2 mts, ¹H chacun : -CH₂- en 14); 2,40 (s, 3H : -COCH₃) ; 3,27 (mt, 1H : -OH en 2') ; 4,04 et 4,32 (2 d, J = 9, 1H chacun : -CH₂- en 20) ; 4,13 (d, J = 7,5, 1H : -H en 3) ; 4,63 (mt, 1H : -H en 2') ; 4,76 (d, J = 4, 1H : -H en 5) ; 5,27 (d large, J = 10z, 1H : -H en 3') ; 5,33 (d, J = 10, 1H : -CONH-) ; 5,72 (d, J = 7,5, 1H : -H en 2) 6,29 (t large, J = 8,5, 1H : -H en 13); 6,58 (s, 1H : -H en 10) ; de 7,25 à 7,45 (mt, 5H : -C₆H₅ en 3') ; 7,53 [(t, J = 7,5, 2H : -OCOC₆H₅(-H en 3 et H en 5)] ; 7,63 [(t, J = 7,5, 1H : -OCOC₆H5(-H en 4)] ; 7,88 [(dd, J = 6 et 1,5, 2H : -OCOC₅H₄N(-H en 3 et -H en 5)] ; 8,17 [(d, J = 7,5, 2H : -OCOC₆H₅(-H en 2 et -H en 6)] ; 8,82 [(dd, J = 6 et 1,5, 2H : -OCOC₅H₄N(-H en 2 et -H en 6)].

Les nouveaux produits de formule générale (I) dans laquelle Z représente un radical de formule générale (II) manifestent une activité inhibitrice significative de la prolifération cellulaire anormale et possèdent des propriétés thérapeutiques permettant le traitement de malades ayant des conditions pathologiques associées à une prolifération cellulaire anormale. Les conditions pathologiques incluent la prolifération cellulaire anormale de cellules malignes ou non malignes de divers tissus et/ou organes, comprenant, de manière non limitative, les tissus musculaires, osseux ou conjonctifs, la peau, le cerveau, les poumons, les organes sexuels, les systèmes lymphatiques ou rénaux, les cellules mammaires ou sanguines, le foie, l'appareil digestif, le pancréas et les glandes thyroïdes ou adrénales. Ces conditions pathologiques peuvent inclure également le psoriasis, les tumeurs solides, les cancers de l'ovaire, du sein, du cerveau, de la prostate, du colon, de l'estomac, du rein ou des testicules, le sarcome de Kaposi, le cholangiocarcinome, le choriocarcinome, le neuroblastome, la tumeur de Wilms, la maladie de Hodgkin, les mélanomes, les myélomes multiples, les leucémies lymphocytaires chroniques, les lymphomes granulocytaires aigus ou chroniques. Les nouveaux produits selon l'invention sont particulièrement utiles pour le traitement du cancer de l'ovaire. Les produits selon l'invention peuvent être utilisés pour prévenir ou retarder l'apparition ou la réapparition des conditions pathologiques ou pour traiter ces conditions pathologiques.

Les produits selon l'invention peuvent être administrés à un malade selon différentes formes adaptées à la voie d'administration choisie qui, de préférence, est la voie parentérale. L'administration par voie parentérale comprend les administrations intraveineuse, intrapéritonéale, intramusculaire ou sous-cutanée. Plus particulièrement préférée est l'administration intrapéritonéale ou intraveineuse.

La présente invention comprend également les compositions pharmaceutiques qui contiennent au moins un produit de formule générale (I) dans laquelle Z représente un radical de formule générale (II) en une quantité suffisante adaptée à l'emploi en thérapeutique humaine ou vétérinaire. Les compositions peuvent être préparées selon les méthodes habituelles en utilisant un ou plusieurs adjuvants, supports ou excipients pharmaceutiquement acceptables. Les supports convenables incluent les diluants, les milieux aqueux stériles et divers solvants non toxiques. De préférence les compositions se présentent sous forme de solutions ou de suspensions aqueuses, de solutions injectables qui peuvent contenir des agents émusifiants, des colorants, des préservatifs ou des stabilisants.

Le choix des adjuvants ou excipients peut être déterminé par la solubilité et les propriétés chimiques du produit, le mode particulier d'administration et les bonnes pratiques pharmaceutiques.

Pour l'administration parentérale, on utilise des solutions ou des suspensions stériles aqueuses ou non aqueuses. Pour la préparation de solutions ou de suspensions non aqueuses peuvent être utilisés des huiles végétales naturelles telle que l'huile d'olive, l'huile de sésame ou l'huile de paraffine ou les esters organiques injectables tel que l'oléate d'éthyle. Les solutions stériles aqueuses peuvent être constituées d'une solution d'un sel pharmaceutiquement acceptable en solution dans de l'eau. Les solutions aqueuses conviennent pour l'administration intraveineuse dans la mesure où le pH est convenablement ajusté et où l'isotonicité est réalisée, par exemple, par une quantité suffisante de chlorure de sodium ou de glucose. La stérélisation peut être réalisée par chauffage ou par tout autre moyen qui n'altère pas la composition.

Il est bien entendu que tous les produits entrant dans les compositions selon l'invention doivent être purs et non toxiques pour les quantités utilisées.

Les compositions peuvent contenir au moins 0,01 % de produit thérapeutiquement actif. La quantité de produit actif dans une composition est telle qu'une posologie convenable puisse être prescrite. De préférence, les compositions sont préparées de telle façon qu'une dose unitaire contienne de 0,01 à 1000 mg environ de produit actif pour l'administration par voie parentérale.

Le traitement thérapeutique peut être effectué concuremment avec d'autres traitements thérapeutiques incluant des médicaments antinéoplastiques, des anticorps monoclonaux, des thérapies immunologiques ou des radiothérapies ou des modificateurs des réponses biologiques. Les modificateurs des réponses incluent, de manière non limitative, les lymphokines et les cytokines telles que les interleukines, les interférons (α, β ou δ) et le TNF. D'autres agents chimiothérapeutiques utiles dans le traitement des désordres dus à la prolifération anormale des cellules incluent, de manière non limitative, les agents alkylants tels que les moutardes à l'azote comme la mechlorethamine, le cyclophosphamide, le melphalan et le chlorambucil, des sulfonates d'alkyle comme le busulfan, les nitrosourées comme la carmustine, la lomustine, la sémustine et la streptozocine, les triazènes comme la dacarbazine, les antimétabolites comme les analogues de l'acide folique tel que le méthotrexate, les analogues de pyrimidine comme le fluorouracil et la cytarabine, des analogues de purines comme la mercaptopurine et la thioguanine, des produits naturels tels que les alcaloïdes de vinca comme la vinblastine, la vincristine et la vindésine, des épipodophyllotoxines comme l'étoposide et le teniposide, des antibiotiques comme la dactinomycine, la daunorubicine, la doxorubicine, la bléomycine, la plicamycine et la mitomycine, des enzymes comme la L-asparaginase, des agents divers comme les complexes de coordination du platine tel que le cisplatine, les urées substituées tel que l'hydroxyurée, les dérivés de méthylhydrazine comme la procarbazine, les suppresseurs adrénocorticoïques comme le mitotane et l'aminoglutéthymide, les hormones et les antagonistes comme les adrénocorticostéroïdes comme la prednisone, les progestines comme le caproate d'hydroxyprogestérone, l'acétate de méthoxyprogestérone et l'acétate de megestrol, les oestrogènes comme le diéthylstilbestrol et l'éthynylestradiol, les antioestrogène comme le tamoxifène, les androgènes comme le propionate de testostérone et la fluoxymesterone.

Les doses utilisées pour mettre en oeuvre les méthodes selon l'invention sont celles qui permettent un traitement prophylactique ou un maximum de réponse thérapeutique. Les doses varient selon la forme d'administration, le produit particulier sélectionné et les caractéristiques propres du sujet à traiter. En général, les doses sont celles qui sont thérapeutiquement efficaces pour le traitement des désordres dus à une prolifération cellulaire anormale. Les produits selon l'invention peuvent être administrés aussi souvent que nécessaire pour obtenir l'effet thérapeutique désiré. Certains malades peuvent répondre rapidement à des doses relativement fortes ou faibles puis avoir besoin de doses d'entretien faibles ou nulles. Généralement, de faibles doses seront utilisées au début du traitement et, si nécessaire, des doses de plus en plus fortes seront administrées jusqu'à l'obtention d'un effet optimum. Pour d'autres malades il peut être nécessaire d'administrer des doses d'entretien 1 à 8 fois par jour, de préférence 1 à 4 fois, selon les besoins physiologiques du malade considéré. Il est aussi possible que pour certains malades il soit nécessaire de n'utiliser qu'une à deux administrations journalières.

Chez l'homme, les doses sont généralement comprises entre 0,01 et 200 mg/kg. Par voie intrapéritonéale, les doses seront en général comprises entre 0,1 et 100 mg/kg et, de préférence entre 0,5 et 50 mg/kg et, encore plus spécifiquement entre 1 et 10 mg/kg. Par voie intraveineuse, les doses sont généralement comprises entre 0,1 et 50 mg/kg et, de préférence entre 0,1 et 5 mg/kg et, encore plus spécifiquement entre 1 et 2 mg/kg. Il est entendu que, pour choisir le dosage le plus approprié, devront être pris en compte la voie d'administration, le poids du malade, son état de santé général, son âge et tous les facteurs qui peuvent influer sur l'efficacité du traitement.

L'exemple suivant illustre une composition selon l'invention.

### EXEMPLE

On dissout 40 mg du produit obtenu à l'exemple 1 dans 1 cm3 d'Emulphor EL 620 et 1 cm3 d'éthanol puis la solution est diluée par addition de 18 cm3 de sérum physiologique.

La composition est administrée par perfusion pendant 1 heure par introduction dans du soluté physiologique.

## Revendications

1. Taxoïdes de formule générale : 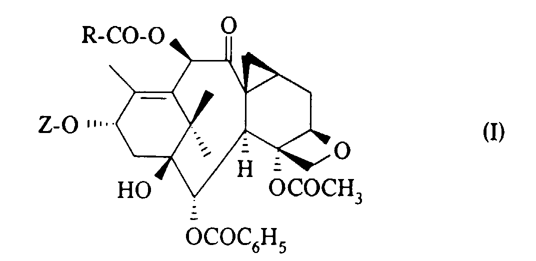 dans laquelle :
R représente
- un radical alcoyle droit ou ramifié contenant 1 à 8 atomes de carbone substitué par un atome d'halogène choisi parmi les atomes de fluor, de chlore, de brome et d'iode ou par un radical amino, alcoylamino dont la partie alcoyle contient 1 à 4 atomes de carbone, dialcoylamino dont chaque partie alcoyle contient 1 à 4 atomes de carbone ou forme avec l'atome d'azote auquel elle est liée un radical hétérocyclique saturé contenant 5 ou 6 chaînons et éventuellement un second hétéroatome choisi parmi les atomes d'oxygène, de soufre ou d'azote (éventuellement substitué par un radical alcoyle contenant 1 à 4 atomes de carbone ou un radical phényle ou un radical phénylalcoyle dont la partie alcoyle contient 1 à 4 atomes de carbone), carboxy, alcoyloxycarbonyle dont la partie alcoyle contient 1 à 4 atomes de carbone, cyano, carbamoyle, N-alcoylcarbamoyle dont la partie alcoyle contient 1 à 4 atomes de carbone ou N,N-dialcoylcarbamoyle dont chaque partie alcoyle contient 1 à 4 atomes de carbone ou forme avec l'atome d'azote auquel elle est liée un radical hétérocyclique saturé contenant 5 ou 6 chaînons et éventuellement un second hétéroatome choisi parmi les atomes d'oxygène, de soufre ou d'azote (éventuellement substitué par un radical alcoyle contenant 1 à 4 atomes de carbone ou un radical phényle ou un radical phénylalcoyle dont la partie alcoyle contient 1 à 4 atomes de carbone),
- un radical alcényle contenant 2 à 8 atomes de carbone en chaîne droite ou ramifiée, un radical alcynyle contenant 2 à 8 atomes de carbone en chaîne droite ou ramifiée, un radical cycloalcoyle contenant 3 à 6 atomes de carbone, cycloalcényle contenant 3 à 6 atomes de carbone, ces radicaux étant éventuellement substitués par un atome d'halogène choisi parmi les atomes de fluor, de chlore, de brome et d'iode ou par un radical amino, alcoylamino dont la partie alcoyle contient 1 à 4 atomes de carbone, dialcoylamino dont chaque partie alcoyle contient 1 à 4 atomes de carbone ou forme avec l'atome d'azote auquel elle est liée un radical hétérocyclique saturé contenant 5 ou 6 chaînons et contenant éventuellement un second hétéroatome choisi parmi les atomes d'oxygène, de soufre ou d'azote (éventuellement substitué par un radical alcoyle contenant 1 à 4 atomes de carbone ou un radical phényle ou un radical phénylalcoyle dont la partie alcoyle contient 1 à 4 atomes de carbone), carboxy, alcoyloxycarbonyle dont la partie alcoyle contient 1 à 4 atomes de carbone, cyano, carbamoyle, N-alcoylcarbamoyle dont la partie alcoyle contient 1 à 4 atomes de carbone ou N,N-dialcoylcarbamoyle dont chaque partie alcoyle contient 1 à 4 atomes de carbone ou forme avec l'atome d'azote auquel elle est liée un radical hétérocyclique saturé contenant 5 ou 6 chaînons et éventuellement un second hétéroatome choisi parmi les atomes d'oxygène, de soufre ou d'azote (éventuellement substitué par un radical alcoyle contenant 1 à 4 atomes de carbone ou un radical phényle ou un radical phénylalcoyle dont la partie alcoyle contient 1 à 4 atomes de carbone), ou
- un radical phényle ou hétérocyclique aromatique à 5 ou 6 chaînons contenant comme hétéroatome un atome d'oxygène, de soufre ou d'azote, et
Z représente un atome d'hydrogène ou un radical de formule générale : 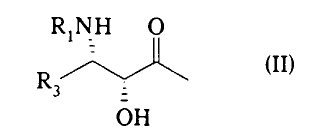 dans laquelle :
R₁ représente un radical benzoyle éventuellement substitué par un ou plusieurs atomes ou radicaux, identiques ou différents, choisis parmi les atomes d'halogène et les radicaux alcoyles contenant 1 à 4 atomes de carbone, alcoxy contenant 1 à 4 atomes de carbone ou trifluorométhyle, thénoyle ou furoyle ou un radical R₂-O-CO- dans lequel R₂ représente :
- un radical alcoyle contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone, bicycloalcoyle contenant 7 à 10 atomes de carbone, ces radicaux étant éventuellement substitués par un ou plusieurs substituants choisis parmi les atomes d'halogène et les radicaux hydroxy, alcoxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle (éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone), cyano, carboxy ou alcoxycarbonyle dont la partie alcoyle contient 1 à 4 atomes de carbone,
- un radical phényle ou α- ou β-naphtyle éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone,
- ou un radical hétérocyclique aromatique à 5 chaînons choisi de préférence parmi les radicaux furyle et thiényle,
- ou un radical hétérocyclyle saturé contenant 4 à 6 atomes de carbone éventuellement substitué par un ou plusieurs radicaux alcoyles contenant 1 à 4 atomes de carbone,
R₃ représente un radical alcoyle droit ou ramifié contenant 1 à 8 atomes de carbone, alcényle droit ou ramifié contenant 2 à 8 atomes de carbone, alcynyle droit ou ramifié contenant 2 à 8 atomes de carbone, cycloalcoyle contenant 3 à 6 atomes de carbone, phényle ou α- ou β-naphtyle éventuellement substitué par un ou plusieurs atomes ou radicaux choisis parmi les atomes d'halogène et les radicaux alcoyles, alcényles, alcynyles, aryles, aralcoyles, alcoxy, alcoylthio, aryloxy, arylthio, hydroxy, hydroxyalcoyle, mercapto, formyle, acyle, acylamino, aroylamino, alcoxycarbonylamino, amino, alcoylamino, dialcoylamino, carboxy, alcoxycarbonyle, carbamoyle, alcoylcarbamoyle, dialcoylcarbamoyle, cyano, nitro et trifluorométhyle, ou un hétérocycle aromatique ayant 5 chaînons et contenant un ou plusieurs hétéroatomes, identiques ou différents, choisis parmi les atomes d'azote, d'oxygène ou de soufre et éventuellement substitué par un ou plusieurs substituants, identiques ou différents, choisis parmi les atomes d'halogène et les radicaux alcoyles, alcoxy, aryles, aryloxy, amino, alcoylamino, dialcoylamino, acylamino, alcoxycarbonylamino, acyle, arylcarbonyle, cyano, carboxy, carbamoyle, alcoylcarbamoyle, dialcoylcarbamoyle ou alcoxycarbonyle, étant entendu que, dans les substituants des radicaux phényle, α- ou β-naphtyle et hétérocyclyles aromatiques, les radicaux alcoyles et les portions alcoyles des autres radicaux contiennent 1 à 4 atomes de carbone et que les radicaux alcényles et alcynyles contiennent 2 à 8 atomes de carbone et que les radicaux aryles sont des radicaux phényles ou α- ou β-naphtyles,

2. Taxoïdes selon la revendication 1 pour lesquels Z représente un atome d'hydrogène ou un radical de formule générale (II) dans laquelle R₁ représente un radical benzoyle ou un radical R₂-O-CO- dans lequel R₂ représente un radical tert-butyle et R₃ représente un radical alcoyle contenant 1 à 6 atomes de carbone, alcényle contenant 2 à 6 atomes de carbone, cycloalcoyle contenant 3 à 6 atomes de carbone, phényle éventuellement substitué par un ou plusieurs atomes ou radicaux, identiques ou différents choisis parmi les atomes d'halogène (fluor, chlore) et les radicaux alcoyles (méthyle), alcoxy (méthoxy), dialcoylamino (diméthylamino), acylamino (acétylamino), alcoxycarbonylamino (tert-butoxycarbonylamino) ou trifluorométhyle ou un radical furyle-2 ou -3, thiényle-2 ou -3 ou thiazolyle-2, -4 ou -5 et R représente un radical cycloalcoyle contenant 3 à 6 atomes de carbone ou un radical phényle, pyridyl-2, -3 ou -4, furyl-2 ou -3 et thiényl-2 ou -3.

3. Taxoïdes selon la revendication 1 pour lesquels Z représente un atome d'hydrogène ou un radical de formule générale (II) dans laquelle R₁ représente un radical benzoyle ou un radical R₂-O-CO- dans lequel R₂ représente un radical tert-butyle et R₃ représente un radical isobutyle, isobutényle, butényle, cyclohexyle, phényle, furyle-2, furyle-3, thiényle-2, thiényle-3, thiazolyle-2, thiazolyle-4 ou thiazolyle-5, R représente un radical cyclopropyle, cyclopentyle, phényle, pyridyle-2, thiényle-2 ou furyle-2.

4. Procédé de préparation d'un produit selon l'une des revendications 1, 2 ou 3 caractérisé en ce que l'on estérifie un produit de formule générale : 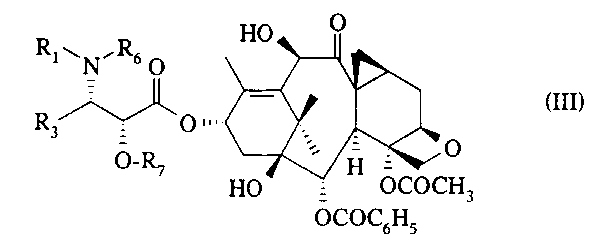 dans laquelle R₁ et R₃ sont définis dans l'une des revendications 1, 2 ou 3, ou bien R₆ représente un atome d'hydrogène et R₇ représente un groupement protecteur de la fonction hydroxy, et ou bien R₆ et R₇ forment ensemble un hétérocycle saturé à 5 ou 6 chaînons, au moyen d'un acide de formule générale :
R-CO-OH (IV)
dans laquelle R est défini comme précédemment, ou d'un dérivé de cet acide tel qu'un halogénure, l'anhydride symétrique ou un anhydride mixte, pour obtenir un produit de formule générale : 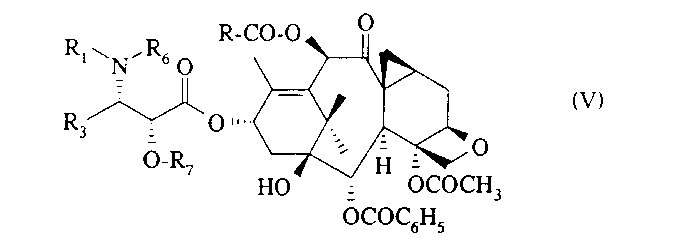 dans laquelle R₁, R₃, R₆ et R₇ sont définis comme précédemment, puis remplace les groupements protecteurs R₇ ou R₆ et R₇ par des atomes d'hydrogène.

5. Procédé selon la revendication 4 caractérisé en ce que l'on effectue l'estérification au moyen d'un acide de formule générale (IV) en présence d'un agent de condensation et d'un agent d'activation dans un solvant organique à une température comprise entre -10 et 90°C.

6. Procédé selon la revendication 4 caractérisé en ce que l'on effectue l'estérification au moyen d'un acide de formule générale (IV) sous forme d'anhydride symétrique en opérant en présence d'un agent d'activation dans un solvant organique à une température comprise entre 0 et 90°C.

7. Procédé selon la revendication 4 caractérisé en ce que l'on effectue l'estérification au moyen d'un acide de formule générale (IV) sous forme d'halogénure ou sous forme d'anhydride mixte avec un acide aliphatique ou aromatique, éventuellement préparé in situ, en présence d'une base en opérant dans un solvant organique à une température comprise entre 0 et 80°C.

8. Procédé selon la revendication 4 caractérisé en ce que l'on effectue le remplacement des groupements protecteurs R₇ et/ou R₆ et R₇ par des atomes d'hydrogène, selon leur nature, en opérant de la manière suivante :
1) lorsque R₆ représente un atome d'hydrogène et R₇ représente un groupement protecteur de la fonction hydroxy, on remplace les groupements protecteurs par des atomes d'hydrogène au moyen d'un acide minéral ou organique utilisé seul ou en mélange en opérant dans un solvant organique choisi parmi les alcools, les éthers, les esters, les hydrocarbures aliphatiques, les hydrocarbures aliphatiques halogénés, les hydrocarbures aromatiques ou les nitriles à une température comprise entre -10 et 60°C,
2) lorsque R₆ et R₇ forment un cycle oxazolidine de formule générale : 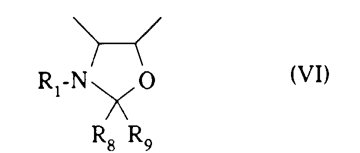 dans laquelle R₁ est défini comme précédemment, R₈ et R₉, identiques ou différents, représentent un atome d'hydrogène ou un radical alcoyle contenant 1 à 4 atomes de carbone, ou un radical aralcoyle dont la partie alcoyle contient 1 à 4 atomes de carbone et la partie aryle représente un radical phényle éventuellement substitué par un ou plusieurs radicaux alcoxy contenant 1 à 4 atomes de carbone, ou un radical aryle représentant un radical phényle éventuellement substitué par un ou plusieurs radicaux alcoxy contenant 1 à 4 atomes de carbone, ou bien R₈ représente un radical alcoxy contenant 1 à 4 atomes de carbone ou un radical trihalométhyle ou un radical phényle substitué par un radical trihalométhyle et R₉ représente un atome d'hydrogène, ou bien R₈ et R₉ forment ensemble avec l'atome de carbone auquel ils sont liés un cycle ayant 4 à 7 chaînons, on remplace le groupement protecteur formé par R₆ et R₇ par des atomes d'hydrogène en opérant, selon les significations de R₁, R₈ et R₉, en opérant de la manière suivante :
a) lorsque R₁ représente un radical tert-butoxycarbonyle, R₈ et R₉, identiques ou différents, représentent un radical alcoyle ou un radical aralcoyle ou aryle, ou bien R₈ représente un radical trihalométhyle ou un radical phényle substitué par un radical trihalométhyle, et R₉ représente un atome d'hydrogène, ou bien R₈ et R₉ forment ensemble un cycle ayant de 4 à 7 chaînons, on traite l'ester de formule générale (V) par un acide minéral ou organique éventuellement dans un solvant organique pour obtenir un produit de formule générale : 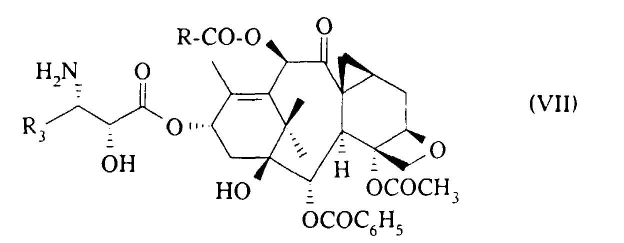 dans laquelle R₃ est défini comme précédemment, que l'on acyle au moyen de chlorure de benzoyle dans lequel le noyau phényle est éventuellemnt substitué, de chlorure de thénoyle, de chlorure de furoyle ou d'un produit de formule générale :
R₂-O-CO-X (VIII)
dans laquelle R₂ est défini comme précédemment et X représente un atome d'halogène ou un reste -O-R₂ ou -O-CO-O-R₂, pour obtenir un produit de formule générale (I) dans laquelle Z représente un radical de formule générale (II).
b) lorsque R₁ représente un radical benzoyle éventuellement substitué, thénoyle ou furoyle ou un radical R₂O-CO- dans lequel R₂ est défini comme précédemment, R₈ représente un atome d'hydrogène ou un radical alcoxy contenant 1 à 4 atomes de carbone ou un radical phényle substitué par un ou plusieurs radicaux alcoxy contenant 1 à 4 atomes de carbone et R₉ représente un atome d'hydrogène, le remplacement du groupement protecteur formé par R₆ et R₇ par des atomes d'hydrogène s'effectue en présence d'un acide minéral ou organique utilisé seul ou en mélange en quantité stoechiométrique ou catalytique, en opérant dans un solvant organique à une température comprise entre -10 et 60°C.

9. Procédé de préparation d'un produit de formule générale (I) dans laquelle Z représente un radical de formule générale (II) caractérisé en ce que l'on estérifie un dérivé de la baccatine III de formule : 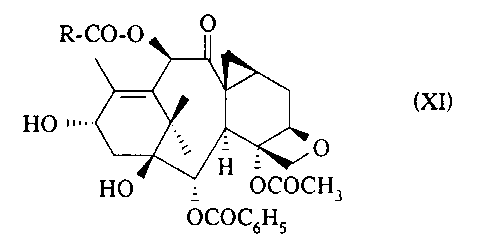 au moyen d'un acide de formule générale : 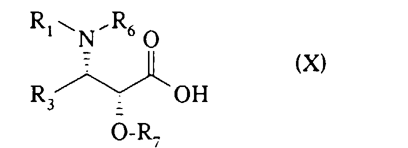 dans laquelle R₁, R₃ sont définis comme dans l'une des revendications 1, 2 ou 3, R₆ et R₇ sont définis comme dans la revendication 4, ou d'un dérivé de cet acide tel qu'un halogénure, l'anhydride symétrique ou un anhydride mixte, suivi du remplacement des groupements protecteurs puis remplace les groupements protecteurs R₇ ou R₆ et R₇ par des atomes d'hydrogène.

10. Procédé selon la revendication 9 caractérisé en ce que l'estérification est effectuée selon le procédé de l'une des revendications 5, 6 ou 7.

11. Procédé selon la revendication 9 caractérisé en ce que lon remplace les groupements protecteurs R₇ ou R₆ et R₇ par des atomes d'hydrogène selon les conditions de la revendication 8.

12. Procédé de préparation d'un produit selon la revendication 1 pour lequel Z représente un atome d'hydrogène caractérisé en ce que l'on estérifie un produit de formule : 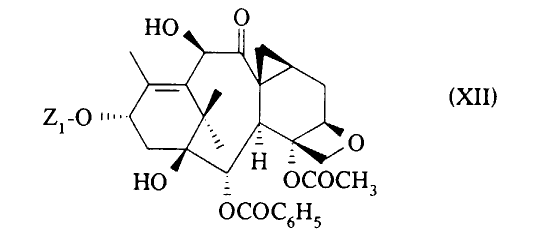 dans laquelle Z₁ représente un groupement protecteur de la fonction hydroxy , au moyen d'un acide de formule générale (IV), ou d'un dérivé de cet acide tel qu'un halogénure ou l'anhydride symétrique ou un anhydride mixte, puis remplace le groupement protecteur Z₁ par un atome d'hydrogène dans des conditions qui ne touchent pas au reste de la molécule.

13. Composition pharmaceutique caractérisée en ce qu'elle contient au moins un produit selon l'une des revendications 1 à 3 pour lequel Z représente un radical de formule générale (II) en association avec un ou plusieurs produits pharmaceutiquement acceptables qu'ils soient inertes ou pharmacologiquement actifs.

## Patentansprüche

1. Taxolderivate der allgemeinen Formel: 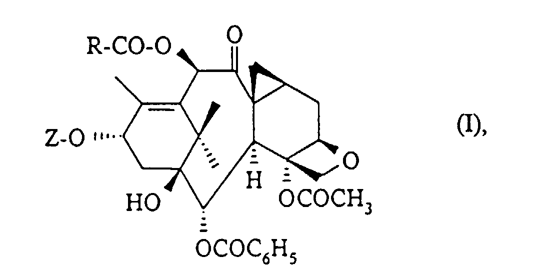 in der R einen der folgenden Reste darstellt:
- einen geradkettigen oder verzweigten Alkylrest, der 1 bis 8 Kohlenstoffatome aufweist und der substituiert ist mit einem unter dem Fluoratom, dem Chloratom, dem Bromatom und dem Iodatom ausgewählten Halogenatom, einem Aminrest, einem Alkylaminrest, dessen Alkylfragment entweder 1 bis 4 Kohlenstoffatome aufweist oder mit dem Stickstoffatom, an das es gebunden ist, einen 5- oder 6-gliedrigen, gesättigten heterozyklischen Rest bildet, der gegebenenfalls ein unter dem Sauerstoffatom, dem Schwefelatom und dem Stickstoffatom (das gegebenenfalls mit einem Alkylrest, der 1 bis 4 Kohlenstoffatome aufweist, einem Phenylrest oder einem Phenylalkylrest, dessen Alkylfragment 1 bis 4 Kohlenstoffatome aufweist, substituiert ist) ausgewähltes zweites Heteroatom enthält, Carboxy, Alkyloxycarbonyl, dessen Alkylfragment 1 bis 4 Kohlenstoffatome aufweist, Cyano, Carbamoyl, N-Alkylcarbamoyl, dessen Alkylfragment 1 bis 4 Kohlenstoffatome aufweist, oder N,N-Dialkylcarbamoyl, dessen Alkylfragmente jeweils 1 bis 4 Kohlenstoffatome aufweisen oder mit dem Stickstoffatom, an das sie gebunden sind, einen 5- oder 6-gliedrigen, gesättigten heterozyklischen Rest bilden, der gegebenenfalls ein unter dem Sauerstoffatom, dem Schwefelatom und dem Stickstoffatom (das gegebenenfalls mit einem Alkylrest, der 1 bis 4 Kohlenstoffatome aufweist, einem Phenylrest oder einem Phenylalkylrest, dessen Alkylfragment 1 bis 4 Kohlenstoffatome aufweist, substituiert ist) ausgewähltes zweites Heteroatom enthält,
- einen Alkenylrest, der in einer geradkettigen oder verzweigten Kette 2 bis 8 Kohlenstoffatome aufweist, einen Alkinylrest, der in einer geradkettigen oder verzweigten Kette 2 bis 8 Kohlenstoffatome aufweist, einen Cycloalkylrest, der 3 bis 6 Kohlenstoffatome aufweist, oder einen Cycloalkenylrest, der 3 bis 6 Kohlenstoffatome aufweist, wobei diese Reste gegebenenfalls mit einem unter dem Fluoratom, dem Chloratom, dem Bromatom und dem Iodatom ausgewählten Halogenatom, einem Aminrest, einem Alkylaminrest, dessen Alkylfragment entweder 1 bis 4 Kohlenstoffatome aufweist oder mit dem Stickstoffatom, an das es gebunden ist, einen 5- oder 6-gliedrigen, gesättigten heterozyklischen Rest bildet, der gegebenenfalls ein unter dem Sauerstoffatom, dem Schwefelatom und dem Stickstoffatom (das gegebenenfalls mit einem Alkylrest, der 1 bis 4 Kohlenstoffatome aufweist, einem Phenylrest oder einem Phenylalkylrest, dessen Alkylfragment 1 bis 4 Kohlenstoffatome aufweist, substituiert ist) ausgewähltes zweites Heteroatom enthält, Carboxy, Alkyloxycarbonyl, dessen Alkylfragment 1 bis 4 Kohlenstoffatome aufweist, Cyano, Carbamoyl, N-Alkylcarbamoyl, dessen Alkylfragment 1 bis 4 Kohlenstoffatome aufweist, oder N,N-Dialkylcarbamoyl, dessen Alkylfragmente jeweils 1 bis 4 Kohlenstoffatome aufweisen oder mit dem Stickstoffatom, an das sie gebunden sind, einen 5- oder 6-gliedrigen, gesättigten heterozyklischen Rest bilden, der gegebenenfalls ein unter dem Sauerstoffatom, dem Schwefelatom und dem Stickstoffatom (das gegebenenfalls mit einem Alkylrest, der 1 bis 4 Kohlenstoffatome aufweist, einem Phenylrest oder einem Phenylalkylrest, dessen Alkylfragment 1 bis 4 Kohlenstoffatome aufweist, substituiert ist) ausgewähltes zweites Heteroatom enthält,
oder
- einen Phenylrest oder einen 5- oder 6-gliedrigen, heterozyklischen aromatischen Rest, der als Heteroatom ein Sauerstoffatom, ein Schwefelatom oder ein Stickstoffatom enthält,
und in der Z ein Wasserstoffatom oder einen Rest der allgemeinen Formel: 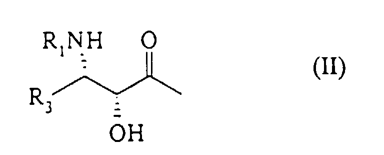 darstellt, in der:
- R₁ einen gegebenenfalls mit einem oder mehreren gleichen oder verschiedenen Atomen oder Resten substituierten Benzoylrest darstellt, wobei diese Atome beziehungsweise Reste ausgewählt sind unter den Halogenatomen, den Alkylresten, die 1 bis 4 Kohlenstoffatome aufweisen, den Alkoxyresten, die 1 bis 4 Kohlenstoffatome aufweisen, dem Trifluormethylrest, dem Thienoylrest, dem Furoylrest und dem Rest R₂-O-CO-, in dem R₂ einen der folgenden Reste darstellt:
- einen Alkylrest, der 1 bis 8 Kohlenstoffatome aufweist, einen Alkenylrest, der 2 bis 8 Kohlenstoffatome aufweist, einen Alkinylrest, der 3 bis 8 Kohlenstoffatome aufweist, einen Cycloalkylrest, der 3 bis 6 Kohlenstoffatome aufweist, einen Cycloalkenylrest, der 4 bis 6 Kohlenstoffatome aufweist, oder einen Bicycloalkylrest, der 7 bis 10 Kohlenstoffatome aufweist, wobei diese Reste gegebenenfalls mit einem oder mehreren Substituenten substituiert sind, die unter den Halogenatomen, dem Hydroxylrest, den Alkoxyresten, die 1 bis 4 Kohlenstoffatome aufweisen, den Dialkylaminoresten, deren Alkylfragmente jeweils 1 bis 4 Kohlenstoffatome aufweisen, Piperidino, Morpholino, Piperazinyl-1 (das gegebenenfalls in Position 4 mit einem Alkylrest, der 1 bis 4 Kohlenstoffatome aufweist, oder einem Phenylalkylrest, dessen Alkylfragment 1 bis 4 Kohlenstoffatome aufweist, substituiert ist), den Cycloalkylresten, die 3 bis 6 Kohlenstoffatome aufweisen, den Cycloalkenylresten, die 4 bis 6 Kohlenstoffatome aufweisen, und dem Phenylrest (der gegebenenfalls mit einem oder mehreren unter den Halogenatomen, den Alkylresten, die 1 bis 4 Kohlenstoffatome aufweisen, den Alkoxyresten, die 1 bis 4 Kohlenstoffatome aufweisen, dem Cyanrest, dem Carboxylrest und den Alkoxycarbonylresten, deren Alkylfragment 1 bis 4 Kohlenstoffatome aufweist, ausgewählten Atomen oder Resten substituiert ist) ausgewählt sind,
- einen Phenyl- oder α- oder β-Naphthylrest, der gegebenenfalls mit einem oder mehreren unter den Halogenatomen, den Alkylresten, die 1 bis 4 Kohlenstoffatome aufweisen, und den Alkoxyresten, die 1 bis 4 Kohlenstoffatome aufweisen, ausgewählten Atomen oder Resten substituiert ist,
- einen 5-gliedrigen, aromatischen heterozyklischen Rest, der vorzugsweise unter dem Furylrest und dem Thienylrest ausgewählt ist,
oder
- einen gesättigten heterozyklischen Rest, der 4 bis 6 Kohlenstoffatome aufweist und der gegebenenfalls mit einem oder mehreren Alkylresten, die 1 bis 4 Kohlenstoffatome aufweisen, substituiert ist,
und
- R₃ einen geradkettigen oder verzweigten Alkylrest, der 1 bis 8 Kohlenstoffatome aufweist, einen geradkettigen oder verzweigten Alkenylrest, der 2 bis 8 Kohlenstoffatome aufweist, einen geradkettigen oder verzweigten Alkinylrest, der 2 bis 8 Kohlenstoffatome aufweist, einen Cycloalkylrest, der 3 bis 6 Kohlenstoffatome aufweist, einen Phenyl- oder α- oder β-Naphthylrest, der gegebenenfalls mit einem oder mehreren unter den Halogenatomen und den Alkyl-, Alkenyl-, Alkinyl-, Aryl-, Arylalkyl-, Alkoxy-, Alkylthio-, Aryloxy-, Arylthio-, Hydroxyl-, Hydroxyalkyl-, Mercapto-, Formyl-, Acyl-, Acylamino-, Arylamino-, Alkoxycarbonylamino-, Amino-, Alkylamino-, Dialkylamino-, Carboxyl-, Alkoxycarbonyl-, Carbamoyl-, Alkylcarbamoyl-, Dialkylcarbamoyl-, Cyan-, Nitro- und Trifluormethylresten ausgewählten Atomen oder Resten substituiert ist, einen 5-gliedrigen, aromatischen heterozyklischen Rest, der ein oder mehrere gleiche oder verschiedene unter dem Stickstoffatom, dem Sauerstoffatom und dem Schwefelatom ausgewählte Heteroatome aufweist und der gegebenenfalls mit einem oder mehreren gleichen oder verschiedenen unter den Halogenatomen und den Alkyl-, Alkoxy-, Aryl-, Aryloxy-, Amino-, Alkylamino-, Dialkylamino-, Acylamino-, Alkoxycarbonylamino-, Acyl-, Arylcarbonyl-, Cyan-, Carboxyl-, Carbamoyl-, Alkylcarbamoyl-, Dialkylcarbamoyl- und Alkoxycarbamoylresten ausgewählten Substituenten substituiert ist,
mit der Maßgabe, daß in den Substituenten der Phenylreste, der α- oder β-Naphthylreste und der aromatischen heterozyklischen Reste die Alkylreste sowie die Alkylfragmente der anderen Reste 1 bis 4 Kohlenstoffeatome aufweisen, daß die Alkenyl- und Alkinylreste 2 bis 8 Kohlenstoffatome aufweisen und daß die Arylreste Phenyl- oder α- oder β-Naphthylreste sind.

2. Taxolderivate nach Anspruch 1, in denen Z ein Wasserstoffatom oder einen Rest der allgemeinen Formel (II) darstellt, wobei in dieser Formel (II) R₁ einen Benzoylrest oder einen Rest R₂-O-CO- darstellt, in welchem R₂ einen tert.-Butylrest bezeichnet, und R₃ einen Alkylest, der 1 bis 6 Kohlenstoffatome aufweist, einen Alkenylrest, der 2 bis 6 Kohlenstoffatome aufweist, einen Cycloalkylrest, der 3 bis 6 Kohlenstoffatome aufweist, einen gegebenenfalls mit einem oder mehreren gleichen oder verschiedenen unter den Halogenatomen (Fluor, Chlor), den Alkylresten (Methyl), den Alkoxyresten (Methoxy), den Dialkylaminoresten (Dimethylamino), den Acylaminoresten (Acetylamino) und den Alkoxycarbonylaminoresten (tert.-Butylcarbonylamino) ausgewählten Atomen oder Resten substituierten Phenylrest, Trifluormethyl oder einen Furyl-2-, Furyl-3-, Thienyl-2-, Thienyl-3-, Thiazolyl-2-, Thiazolyl-4- oder Thiazolyl-5-Rest darstellt, und in denen R einen Cycloalkylrest, der 3 bis 6 Kohlenstoffatome aufweist, einen Phenylrest oder einen Pyridyl-2-, Pyridyl-3-, Pyridyl-4-, Furyl-2-, Furyl-3-, Thienyl-2- oder Thienyl-3-Rest darstellt.

3. Taxolderivate nach Anspruch 1, in denen Z ein Wasserstoffatom oder einen Rest der allgemeinen Formel (II) darstellt, wobei in dieser Formel (II) R₁ einen Benzoylrest oder einen Rest R₂-O-CO- darstellt, in welchem R₂ einen tert.-Butylrest bezeichnet, und R₃ einen Isobutyl-, Isobutenyl-, Butenyl-, Cyclohexyl-, Phenyl-, Furyl-2-, Furyl-3-, Thienyl-2-, Thienyl-3-, Thiazolyl-2-, Thiazolyl-4- oder Thiazolyl-5-Rest darstellt, und in denen R einen Cyclopropyl-, Cyclopentyl-, Phenyl-, Pyridyl-2-, Thienyl-2- oder Furyl-2-Rest darstellt.

4. Verfahren zur Herstellung einer Verbindung nach einem der Ansprüche 1, 2 und 3, dadurch gekennzeichnet, daß eine Verbindung der allgemeinen Formel: 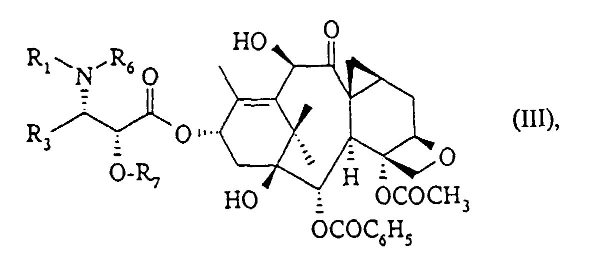 wobei R₁ und R₃ in einem der Ansprüche 1, 2 und 3 definiert sind und wobei in dieser Formel (III) R₆ ein Wasserstoffatom und R₇ eine Schutzgruppe für die Hydroxylfunktion darstellt oder R₆ und R₇ gemeinsam einen 5- oder 6-gliedrigen gesättigten Heterozyklus bilden,
mit einer Säure der allgemeinen Formel:
R-CO-OH (IV),
in der R die oben angegebene Bedeutung hat,
oder einem Derivat einer solchen Säure, wie einem Halogenid oder einem symmetrischen oder gemischten Anhydrid, unter Erhalt einer Verbindung der allgemeinen Formel: 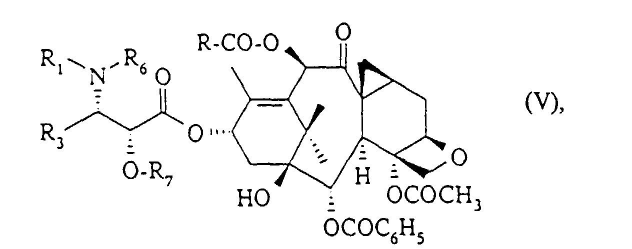 in der R₁, R₃, R₆ und R₇ die oben angegebene Bedeutung haben,
verestert wird und die Schutzgruppen R₇ beziehungsweise R₆ und R₇ anschließend durch Wasserstoffatome ersetzt werden.

5. Verfahren nach Anspruch 4, dadurch gekennzeichnet, daß eine Veresterung mit einer Säure der allgemeinen Formel (IV) in Gegenwart eines Kondensationsmittels und eines Aktivierungsmittels in einem organischen Lösungsmittel bei einer Temperatur im Bereich von -10 bis 90 °C durchgeführt wird.

6. Verfahren nach Anspruch 4, dadurch gekennzeichnet, daß die Veresterung mit einer in Form des symmetrischen Anhydrids vorliegenden Säure der allgemeinen Formel (IV) durchgeführt wird, wobei in Gegenwart eines Aktivierungsmittels in einem organischen Lösungsmittel bei einer Temperatur im Bereich von 0 bis 90 °C gearbeitet wird.

7. Verfahren nach Anspruch 4, dadurch gekennzeichnet, daß die Veresterung mit einer Säure der allgemeinen Formel (IV) durchgeführt wird, die in Form eines Halogenids oder eines gegebenenfalls in situ hergestellten gemischten Anhydrids mit einer aliphatischen oder aromatischen Säure vorliegt, wobei in Gegenwart einer Base in einem organischen Lösungsmittel bei einer Temperatur im Bereich von 0 bis 80 °C gearbeitet wird.

8. Verfahren nach Anspruch 4, dadurch gekennzeichnet, daß das Ersetzen der Schutzgruppen R₇ und/oder R₆ und R₇ durch Wasserstoffatome in Abhängigkeit von der Art dieser Schutzgruppen auf folgende Weise durchgeführt wird:
1) stellt R₆ ein Wasserstoffatom dar und R₇ eine Schutzgruppe für die Hydroxylfunktion, so werden die Schutzgruppen mit Hilfe einer mineralischen oder organischen Säure, die als Einzelsubstanz oder in Form eines Gemischs eingesetzt wird, durch Wasserstoffatome ersetzt, wobei in einem unter den Alkoholen, den Ethern, den Estern, den aliphatischen Kohlenwasserstoffen, den halogenierten aliphatischen Kohlenwasserstoffen, den aromatischen Kohlenwasserstoffen und den Nitrilen ausgewählten organischen Lösungsmittel bei einer Temperatur im Bereich von -10 bis 60 °C gearbeitet wird,
2) bilden R₆ und R₇ einen Oxazolidin-Ring der allgemeinen Formel: 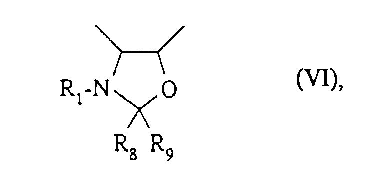 in der R₁ die oben angegebene Bedeutung hat und in der R₈ und R₉, die gleich oder verschieden sein können, Wasserstoffatome, Alkylreste, die 1 bis 4 Kohlenstoffatome aufweisen, Arylalkylreste, deren Alkylfragment 1 bis 4 Kohlenstoffatome aufweist und deren Arylfragment ein gegebenenfalls mit einem oder mehreren Alkoxyresten, die 1 bis 4 Kohlenstoffatome aufweisen, substituierter Phenylrest ist, oder Arylreste darstellen, die aus einem gegebenenfalls mit einem oder mehreren Alkoxyresten, die 1 bis 4 Kohlenstoffatome aufweisen, substituierten Phenylrest bestehen, oder in der R₈ einen Alkoxyrest, der 1 bis 4 Kohlenstoffatome aufweist, einen dreifach halogenierten Methylrest oder einen mit einem dreifach halogenierten Methylrest substituierten Phenylrest und R₉ ein Wasserstoffatom darstellt oder in der R₈ und R₉ zusammen mit dem Kohlenstoffatom, an das sie gebunden sind, einen 4- bis 7-gliedrigen Ring bilden,
so wird die durch R₆ und R₇ gebildete Schutzgruppe durch Wasserstoffatome ersetzt, indem in Abhängigkeit von den Resten R₁, R₈ und R₉ folgendermaßen vorgegangen wird:
a) bezeichnet R₁ einen tert.-Butoxycarbonylrest und bezeichnen R₈ und R₉, die gleich oder verschieden sein können, Alkyl-, Arylalkyl- oder Arylreste, oder bezeichnet R₈ einen dreifach halogenierten Methylrest oder einen mit einem dreifach halogenierten Methylrest substituierten Phenylrest und bezeichnet R₉ ein Wasserstoffatom, oder bilden R₈ und R₉ zusammen einen 4- bis 7-gliedrigen Ring, so wird der Ester der allgemeinen Formel (V) - gegebenenfalls in einem organischen Lösungsmittel - mit einer mineralischen oder organischen Säure behandelt, wobei eine Verbindung der allgemeinen Formel: 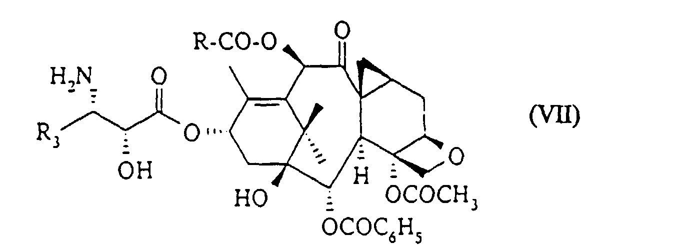 erhalten wird, in der R₃ die oben angegebene Bedeutung hat und die mit Benzoylchlorid, das gegebenenfalls am Phenylring substituiert ist, mit Thienoylchlorid, mit Furoylchlorid oder mit einer Verbindung der allgemeinen Formel:
R₂O-CO-X (VIII),
in der R₂ die oben angegebene Bedeutung hat und X ein Halogenatom oder einen Rest -O-R₂ oder -O-CO-O-R₂ darstellt,
unter Erhalt einer Verbindung der allgemeinen Formel (I), in der Z einen Rest der allgemeinen Formel (II) bezeichnet, acyliert wird,
b) bezeichnet R₁ einen gegebenenfalls substituierten Benzoylrest, einen Thienoyl- oder Furoylrest oder einen Rest R₂O-CO-, in dem R₂ die oben angegebene Bedeutung hat, bezeichnet R₈ ein Wasserstoffatom, einen Alkoxyrest, der 1 bis 4 Kohlenstoffatome aufweist, oder einen mit einem oder mehreren Alkoxyresten, die 1 bis 4 Kohlenstoffatome aufweisen, substituierten Phenylrest und bezeichnet R₉ ein Wasserstoffatom, so erfolgt das Ersetzen der durch R₆ und R₇ gebildeten Schutzgruppe durch Wasserstoffatome in Gegenwart einer mineralischen oder organischen Säure, die als Einzelsubstanz oder in Form eines Gemischs in stöchiometrischer oder katalytischer Menge eingesetzt wird, wobei in einem organischen Lösungsmittel bei einer Temperatur im Bereich von -10 bis 60 °C gearbeitet wird.

9. Verfahren zur Herstellung einer Verbindung der allgemeinen Formel (I), in der Z einen Rest der allgemeinen Formel (II) darstellt, dadurch gekennzeichnet, daß ein Baccatin III-Derivat der Formel: 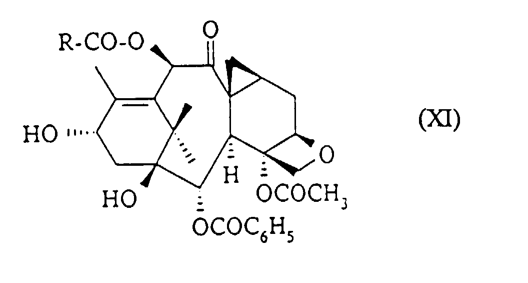 mit einer Säure der allgemeinen Formel: 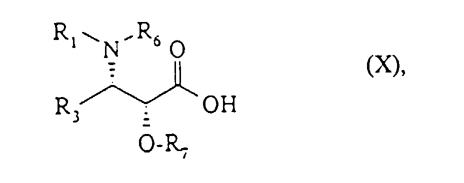 wobei R₁ und R₃ in einem der Ansprüche 1, 2 und 3 definiert sind und wobei in dieser Formel (X) R₆ und R₇ die in Anspruch 4 angegebene Bedeutung haben,
oder einem Derivat einer solchen Säure, wie einem Halogenid oder einem symmetrischen oder gemischten Anhydrid, verestert wird und die Schutzgruppen R₇ beziehungsweise R₆ und R₇ anschließend durch Wasserstoffatome ersetzt werden.

10. Verfahren nach Anspruch 9, dadurch gekennzeichnet, daß die Veresterung nach einem der Verfahren der Ansprüche 5, 6 und 7 durchgeführt wird.

11. Verfahren nach Anspruch 9, dadurch gekennzeichnet, daß die Schutzgruppen R₇ beziehungsweise R₆ und R₇ entsprechend den Bedingungen nach Anspruch 8 durch Wasserstoffatome ersetzt werden.

12. Verfahren zur Herstellung einer Verbindung nach Anspruch 1, in der Z ein Wasserstoffatom darstellt, dadurch gekennzeichnet, daß eine Verbindung der Formel: 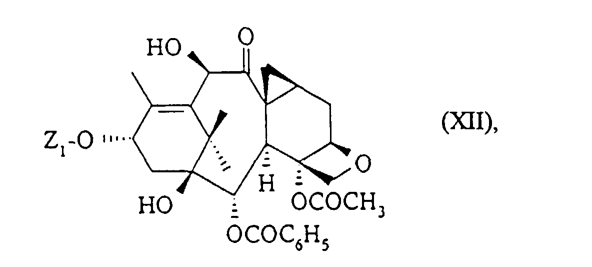 in der Z₁ eine Schutzgruppe für die Hydroxylfunktion darstellt,
mit einer Säure der allgemeinen Formel (IV) oder einem Derivat einer solchen Säure, wie einem Halogenid oder einem symmetrischen oder gemischten Anhydrid, verestert wird und die Schutzgruppe Z₁ anschließend unter Bedingungen, die das übrige Molekül nicht beeinträchtigen, durch ein Wasserstoffatom ersetzt wird.

13. Pharmazeutische Zusammensetzung, dadurch gekennzeichnet, daß sie zumindest eine Verbindung nach einem der Ansprüche 1 bis 3, in der Z einen Rest der allgemeinen Formel (II) darstellt, in Assoziation mit einer oder mehreren pharmazeutisch akzeptablen Ver bindungen, die inert oder pharmakologisch aktiv sein können, enthält.

## Claims

1. Taxoids of general formula: 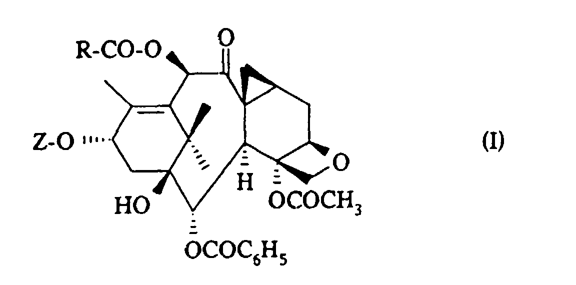 in which:
R represents
- an unbranched or branched alkyl radical containing 1 to 8 carbon atoms, substituted with a halogen atom chosen from fluorine, chlorine, bromine and iodine atoms or with an amino radical, an alkylamino radical in which the alkyl portion contains 1 to 4 carbon atoms, a dialkylamino radical in which each alkyl portion contains 1 to 4 carbon atoms or, with the nitrogen atom to which it is linked, forms a saturated 5- or 6-membered heterocyclic radical optionally containing a second hetero atom chosen from oxygen, sulphur and nitrogen atoms (and optionally substituted with an alkyl radical containing 1 to 4 carbon atoms or a phenyl radical or a phenylalkyl radical in which the alkyl portion contains 1 to 4 carbon atoms), a carboxyl radical, an alkyloxycarbonyl radical in which the alkyl portion contains 1 to 4 carbon atoms, a cyano or carbamoyl radical, an N-alkylcarbamoyl radical in which the alkyl portion contains 1 to 4 carbon atoms or an N,N-dialkylcarbamoyl radical in which each alkyl portion contains 1 to 4 carbon atoms or, with the nitrogen atom to which it is linked, forms a saturated 5- or 6-membered heterocyclic radical optionally containing a second hetero atom chosen from oxygen, sulphur and nitrogen atoms (and optionally substituted with an alkyl radical containing 1 to 4 carbon atoms or a phenyl radical or a phenylalkyl radical in which the alkyl portion contains 1 to 4 carbon atoms),
- an alkenyl radical containing 2 to 8 carbon atoms in an unbranched or branched chain, an alkynyl radical containing 2 to 8 carbon atoms in an unbranched or branched chain, a cycloalkyl radical containing 3 to 6 carbon atoms or a cycloalkenyl radical containing 3 to 6 carbon atoms, these radicals being optionally substituted with a halogen atom chosen from fluorine, chlorine, bromine and iodine atoms or with an amino radical, an alkylamino radical in which the alkyl portion contains 1 to 4 carbon atoms, a dialkylamino radical in which each alkyl portion contains 1 to 4 carbon atoms or, with the nitrogen atom to which it is linked, forms a saturated 5- or 6-membered heterocyclic radical optionally containing a second hetero atom chosen from oxygen, sulphur and nitrogen atoms (and optionally substituted with an alkyl radical containing 1 to 4 carbon atoms or a phenyl radical or a phenylalkyl radical in which the alkyl portion contains 1 to 4 carbon atoms), a carboxyl radical, an alkyloxycarbonyl radical in which the alkyl portion contains 1 to 4 carbon atoms, a cyano or carbamoyl radical, an N-alkylcarbamoyl radical in which the alkyl portion contains 1 to 4 carbon atoms or an N,N-dialkylcarbamoyl radical in which each alkyl portion contains 1 to 4 carbon atoms or, with the nitrogen atom to which it is linked, forms a saturated 5- or 6-membered heterocyclic radical optionally containing a second hetero atom chosen from oxygen, sulphur and nitrogen atoms (and optionally substituted with an alkyl radical containing 1 to 4 carbon atoms or a phenyl radical or a phenylalkyl radical in which the alkyl portion contains 1 to 4 carbon atoms), or
- a phenyl radical or a 5- or 6-membered aromatic heterocyclic radical containing an oxygen, sulphur or nitrogen atom as hetero atom, and
Z represents a hydrogen atom or a radical of general formula: 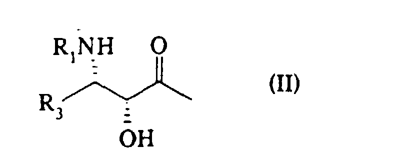 in which:
R₁ represents a benzoyl radical optionally substituted with one or more identical or different atoms or radicals chosen from halogen atoms and alkyl radicals containing 1 to 4 carbon atoms, alkoxy radicals containing 1 to 4 carbon atoms or trifluoromethyl radicals, a thenoyl or furoyl radical or a radical R₂-O-CO- in which R₂ represents:
- an alkyl radical containing 1 to 8 carbon atoms, an alkenyl radical containing 2 to 8 carbon atoms, an alkynyl radical containing 3 to 8 carbon atoms, a cycloalkyl radical containing 3 to 6 carbon atoms, a cycloalkenyl radical containing 4 to 6 carbon atoms or a bicycloalkyl radical containing 7 to 10 carbon atoms, these radicals being optionally substituted with one or more substituents chosen from halogen atoms and hydroxyl radicals, alkoxy radicals containing 1 to 4 carbon atoms, dialkylamino radicals in which each alkyl portion contains 1 to 4 carbon atoms, piperidino or morpholino radicals, 1-piperazinyl radicals (optionally substituted at position 4 with an alkyl radical containing 1 to 4 carbon atoms or with a phenylalkyl radical in which the alkyl portion contains 1 to 4 carbon atoms), cycloalkyl radicals containing 3 to 6 carbon atoms, cycloalkenyl radicals containing 4 to 6 carbon atoms, phenyl radicals (optionally substituted with one or more atoms or radicals chosen from halogen atoms and alkyl radicals containing 1 to 4 carbon atoms or alkoxy radicals containing 1 to 4 carbon atoms), cyano or carboxyl radicals or alkoxycarbonyl radicals in which the alkyl portion contains 1 to 4 carbon atoms,
- a phenyl or α- or β-naphthyl radical optionally substituted with one or more atoms or radicals chosen from halogen atoms and alkyl radicals containing 1 to 4 carbon atoms or alkoxy radicals containing 1 to 4 carbon atoms,
- or a 5-membered aromatic heterocyclic radical preferably chosen from furyl and thienyl radicals,
- or a saturated heterocyclic radical containing 4 to 6 carbon atoms, optionally substituted with one or more alkyl radicals containing 1 to 4 carbon atoms,
R₃ represents an unbranched or branched alkyl radical containing 1 to 8 carbon atoms, an unbranched or branched alkenyl radical containing 2 to 8 carbon atoms, an unbranched or branched alkynyl radical containing 2 to 8 carbon atoms, a cycloalkyl radical containing 3 to 6 carbon atoms, a phenyl or α- or β-naphthyl radical optionally substituted with one or more atoms or radicals chosen from halogen atoms and alkyl, alkenyl, alkynyl, aryl, aralkyl, alkoxy, alkylthio, aryloxy, arylthio, hydroxyl, hydroxyalkyl, mercapto, formyl, acyl, acylamino, aroylamino, alkoxycarbonylamino, amino, alkylamino, dialkylamino, carboxyl, alkoxycarbonyl, carbamoyl, alkylcarbamoyl, dialkylcarbamoyl, cyano, nitro and trifluoromethyl radicals, or a 5-membered aromatic heterocycle containing one or more identical or different hetero atoms chosen from nitrogen, oxygen and sulphur atoms and optionally substituted with one or more identical or different substituents chosen from halogen atoms and alkyl, alkoxy, aryl, aryloxy, amino, alkylamino, dialkylamino, acylamino, alkoxycarbonylamino, acyl, arylcarbonyl, cyano, carboxyl, carbamoyl, alkylcarbamoyl, dialkylcarbamoyl or alkoxycarbonyl radicals, on the understanding that, in the substituents of the phenyl, α- or β-naphthyl and aromatic heterocyclic radicals, the alkyl radicals and the alkyl portions of the other radicals contain 1 to 4 carbon atoms, and that the alkenyl and alkynyl radicals contain 2 to 8 carbon atoms, and that the aryl radicals are phenyl or α- or β-naphthyl radicals.

2. Taxoids according to claim 1 for which Z represents a hydrogen atom or a radical of general formula (II) in which R₁ represents a benzoyl radical or a radical R₂-O-CO- in which R₂ represents a tert-butyl radical and R₃ represents an alkyl radical containing 1 to 6 carbon atoms, an alkenyl radical containing 2 to 6 carbon atoms, a cycloalkyl radical containing 3 to 6 carbon atoms, a phenyl radical optionally substituted with one or more identical or different atoms or radicals chosen from halogen atoms (fluorine, chlorine) and alkyl (methyl), alkoxy (methoxy), dialkylamino (dimethylamino), acylamino (acetylamino), alkoxycarbonylamino (tert-butoxycarbonylamino) or trifluoromethyl radicals, or a 2- or 3-furyl, 2- or 3-thienyl or 2-, 4- or 5-thiazolyl radical, and R represents a cycloalkyl radical containing 3 to 6 carbon atoms or a phenyl, 2-, 3- or 4-pyridyl, 2- or 3-furyl and 2- or 3-thienyl radical.

3. Taxoids according to claim 1 for which Z represents a hydrogen atom or a radical of general formula (II) in which R₁ represents a benzoyl radical or a radical R₂-O-CO- in which R₂ represents a tert-butyl radical and R₃ represents an isobutyl, isobutenyl, butenyl, cyclohexyl, phenyl, 2-furyl, 3-furyl, 2-thienyl, 3-thienyl, 2-thiazolyl, 4-thiazolyl or 5-thiazolyl radical, and R represents a cyclopropyl, cyclopentyl, phenyl, 2-pyridyl, 2-thienyl or 2-furyl radical.

4. Process for preparing a product according to one of claims 1, 2 and 3, characterized in that a product of general formula: 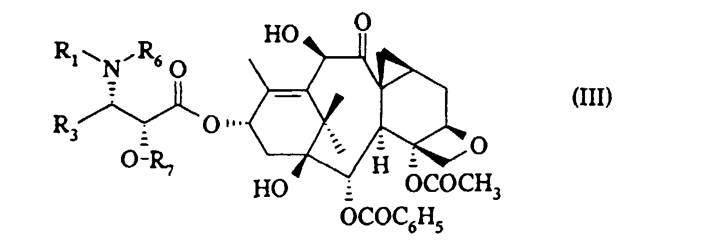 in which R₁ and R₃ are defined in one of claims 1, 2 and 3, and either R₆ represents a hydrogen atom and R₇ represents a group protecting the hydroxyl function, or R₆ and R₇ together form a saturated 5- or 6-membered heterocycle, is esterified by means of an acid of general formula:
R-CO-OH (IV)
in which R is defined as above, or by means of a derivative of this acid, such as a halide, the symmetrical anhydride or a mixed anhydride, to obtain a product of general formula: 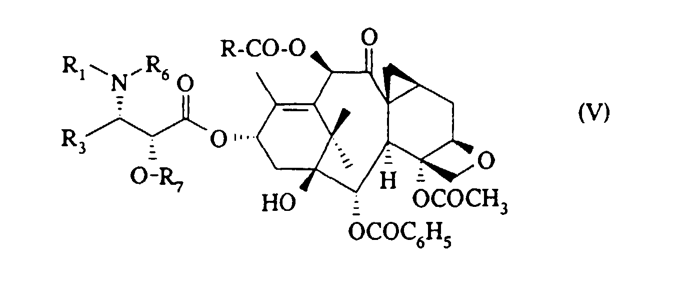 in which R₁, R₃, R₆ and R₇ are defined as above, and the protective groups R₇ or R₆ and R₇ are then replaced by hydrogen atoms.

5. Process according to claim 4, characterized in that the esterification is performed by means of an acid of general formula (IV) in the presence of a condensing agent and an activating agent in an organic solvent at a temperature of between -10 and 90°C.

6. Process according to claim 4, characterized in that the esterification is performed by means of an acid of general formula (IV) in the form of the symmetrical anhydride, working in the presence of an activating agent in an organic solvent at a temperature of between 0 and 90°C.

7. Process according to claim 4, characterized in that the esterification is performed by means of an acid of general formula (IV) in halide form or in the form of a mixed anhydride with an aliphatic or aromatic acid, optionally prepared in situ, in the presence of a base, working in an organic solvent at a temperature of between 0 and 80°C.

8. Process according to claim 4, characterized in that replacement of the protective groups R₇ and/or R₆ and R₇ by hydrogen atoms is performed, working, depending on their nature, in the following manner:
1) when R₆ represents a hydrogen atom and R₇ represents a group protecting the hydroxyl function, the protective groups are replaced by hydrogen atoms by means of an inorganic or organic acid used alone or mixed, working in an organic solvent chosen from alcohols, ethers, esters, aliphatic hydrocarbons, halogenated aliphatic hydrocarbons, aromatic hydrocarbons or nitriles at a temperature of between -10 and 60°C,
2) when R₆ and R₇ form an oxazolidine ring of general formula: 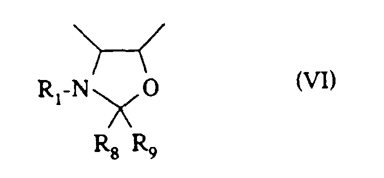 in which R₁ is defined as above and R₈ and R₉, which may be identical or different, represent a hydrogen atom or an alkyl radical containing 1 to 4 carbon atoms, or an aralkyl radical in which the alkyl portion contains 1 to 4 carbon atoms and the aryl portion represents a phenyl radical optionally substituted with one or more alkoxy radicals containing 1 to 4 carbon atoms, or an aryl radical representing a phenyl radical optionally substituted with one or more alkoxy radicals containing 1 to 4 carbon atoms, or alternatively R₈ represents an alkoxy radical containing 1 to 4 carbon atoms or a trihalomethyl radical or a phenyl radical substituted with a trihalomethyl radical and R₉ represents a hydrogen atom, or alternatively R₈ and R₉, together with the carbon atom to which they are linked, form a 4- to 7-membered ring, the protective group formed by R₆ and R₇ is replaced by hydrogen atoms, working, depending on the meanings of R₁, R₈ and R₉, in the following manner:
a) when R₁ represents a tert-butoxycarbonyl radical and R₈ and R₉, which may be identical or different, represent an alkyl radical or an aralkyl or aryl radical, or alternatively R₈ represents a trihalomethyl radical or a phenyl radical substituted with a trihalomethyl radical and R₉ represents a hydrogen atom, or alternatively R₈ and R₉ together form a 4- to 7-membered ring, the ester of general formula (V) is treated with an inorganic or organic acid, where appropriate in an organic solvent, to obtain a product of general formula: 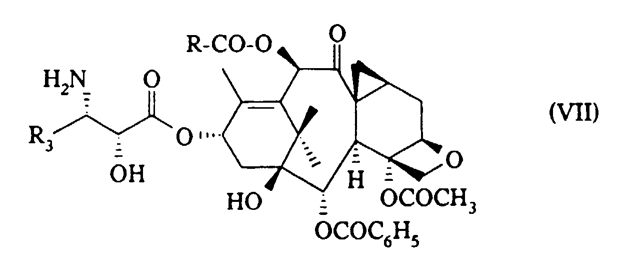 in which R₃ is defined as above, which is acylated by means of benzoyl chloride in which the phenyl ring is optionally substituted or by means of thenoyl chloride, of furoyl chloride or of a product of general formula:
R₂-O-CO-X (VIII)
in which R₂ is defined as above and X represents a halogen atom or a residue -O-R₂ or -O-CO-O-R₂, to obtain a product of general formula (I) in which Z represents a radical of general formula (II),
b) when R₁ represents an optionally substituted benzoyl radical, a thenoyl or furoyl radical or a radical R₂O-CO- in which R₂ is defined as above, R₈ represents a hydrogen atom or an alkoxy radical containing 1 to 4 carbon atoms or a phenyl radical substituted with one or more alkoxy radicals containing 1 to 4 carbon atoms and R₉ represents a hydrogen atom, replacement of the protective group formed by R₆ and R₇ by hydrogen atoms is performed in the presence of an inorganic or organic acid used alone or mixed in a stoichiometric or catalytic amount, working in an organic solvent at a temperature of between -10 and 60°C.

9. Process for preparing a product of general formula (I) in which Z represents a radical of general formula (II), characterized in that a baccatin III derivative of formula: 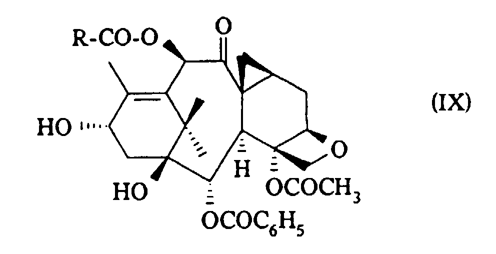 is esterified by means of an acid of general formula: 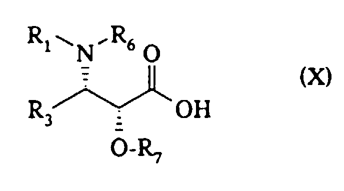 in which R₁ and R₃ are defined as in one of claims 1, 2 and 3, and R₆ and R₇ are defined as in claim 4, or by means of a derivative of this acid such as a halide, the symmetrical anhydride or a mixed anhydride, followed by replacement of the protective groups, and the protective groups R₇ or R₆ and R₇ are then replaced by hydrogen atoms.

10. Process according to claim 9, characterized in that the esterification is performed according to the process of one of claims 5, 6 and 7.

11. Process according to claim 9, characterized in that the protective groups R₇ or R₆ and R₇ are replaced by hydrogen atoms according to the conditions of claim 8.

12. Process for preparing a product according to claim 1 for which Z represents a hydrogen atom, characterized in that a product of formula: 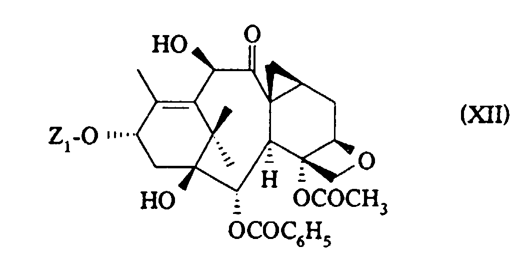 in which Z₁ represents a group protecting the hydroxyl function, is esterified by means of an acid of general formula (IV) or of a derivative of this acid such as a halide or the symmetrical anhydride or a mixed anhydride, and the protective group Z₁ is then replaced by a hydrogen atom under conditions which do not affect the remainder of the molecule.

13. Pharmaceutical composition, characterized in that it contains at least one product according to one of claims 1 to 3 for which Z represents a radical of general formula (II), in combination with one or more pharmaceutically acceptable products, which may be inert or pharmacologically active.
